(19) **Europäisches Patentamt**
**European Patent Office**
**Office européen des brevets**

(11) **EP 2 473 939 B1**

(12) **EUROPEAN PATENT SPECIFICATION**

(45) Date of publication and mention
of the grant of the patent:
**25.01.2017 Bulletin 2017/04**

(21) Application number: **09782656.4**

(22) Date of filing: **05.09.2009**

(51) Int Cl.:
***G06F 19/20*** (2011.01)

(86) International application number:
**PCT/EP2009/061510**

(87) International publication number:
**WO 2011/035801 (31.03.2011 Gazette 2011/13)**

(54) **METHODS AND SYSTEMS FOR ANALYSING HYBRIDISATION**

VERFAHREN UND SYSTEME ZUR HYBRIDISIERUNGSANALYSE

PROCÉDÉS ET SYSTÈMES D'ANALYSE D'HYBRIDATION

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(43) Date of publication of application:
**11.07.2012 Bulletin 2012/28**

(73) Proprietor: **VITO NV (Vlaamse Instelling voor
Technologisch
Onderzoek NV)
2400 Mol (BE)**

(72) Inventors:
• **HOOYBERGHS, Jef
2400 Mol (BE)**
• **CARLON, Enrico
3001 Heverlee (BE)**

(74) Representative: **Paemen, Liesbet R.J. et al
De Clercq & Partners
Edgard Gevaertdreef 10 a
9830 Sint-Martens-Latem (BE)**

(56) References cited:
• **FISH DANIEL J ET AL: "DNA multiplex
hybridization on microarrays and
thermodynamic stability in solution: a direct
comparison." NUCLEIC ACIDS RESEARCH 2007,
vol. 35, no. 21, 18 October 2007 (2007-10-18),
pages 7197-7208, XP002575815 ISSN: 1362-4962**
• **WEI CHENG-WEY ET AL: "Using a microfluidic
device for 1 microl DNA microarray hybridization
in 500 s." NUCLEIC ACIDS RESEARCH 2005,
[Online] vol. 33, no. 8, 12 May 2005 (2005-05-12),
pages 1-11, XP002575816 ISSN: 1362-4962
Retrieved from the Internet:
URL:http://nar.oxfordjournals.org/cgi/scre
enpdf/33/8/e78?maxtoshow=&hits=10&RESULT
FO
RMAT=1&author1=wei&author2=young&title=mi
c rofluidic&andorexacttitle=and&andorexacti
tleabs=and&andorexactfulltext=and&searchid
=1&FIRSTINDEX=0&sortspec=relevance&volum
e= 33&resourcetype=HWCIT> [retrieved on
2010-03-25]**
• **BISHOP J ET AL: "Kinetics of multiplex
hybridization: mechanisms and implications."
BIOPHYSICAL JOURNAL 1 MAR 2008, vol. 94, no.
5, 1 March 2008 (2008-03-01), pages 1726-1734,
XP002575817 ISSN: 1542-0086**

EP 2 473 939 B1

**Description**

**Field of the invention**

The invention relates to the field of microarray analysis. More particularly, the present invention relates to methods and systems for analysis of hybridization, e.g. hybridization between a nucleic acid strand in solution and a complementary strand linked to a solid surface such as hybridisation in microarrays.

**Background of the invention**

Microarrays, such as DNA microarrays, are widely used in the current research in molecular biology. The devices have several important applications as for example in gene expression profiling, in the detection of single nucleotide polymorphisms, in the analysis of copy number variations, etc. What all DNA microarrays have in common is the basic underlying reaction of hybridisation between a nucleic acid strand in solution (target) and a complementary strand linked covalently at a solid surface (probe).

Hybridisation is characterized by a (sequence dependent) free energy difference $\Delta G$ which measures the binding affinity for the two strands to form a duplex. In current microarray experiments it is assumed that the hybridization reaction has reached equilibrium when the optical read-out is performed. According to equilibrium thermodynamics the intensity I from a microarray spot is given by:

$$I = A \; \frac{c.e^{-\Delta G/RT}}{1 + c.e^{-\Delta G/RT}} \qquad\qquad [1]$$

with T the experimental temperature, R the gas constant, c the concentration of the target in solution, A an amplification factor of the optical reading device, and $\Delta G$ the free energy difference between the bound probe-target state (double stranded) and its unbound state.

This theory assumes that the hybridization process is a two-state process: either hybridisation has taken place (bound state) or it has not (unbound state). This approximation is expected to be good for short sequences. In the limit $c.e^{-\Delta G/RT} << 1$ the expression can be simplified to

$$\log(I) = cte + \log(c) - \frac{\Delta G}{RT} \qquad\qquad [2]$$

To exploit this theory in technical applications, and thus to use it for analysis purposes, it is of central importance to have good estimates of $\Delta G$ for a given probe and target sequence. In the past decades, the static and dynamic properties of hybridisation between two floating strands has been discussed. Nearest neighbour models provide reasonable approximation of the free energy difference for strands hybridisation in solution. Such models estimate the hybridization free energy as a sum of dinucleotide parameters. These parameters were fitted through a series of (labour intensive) experiments.

The relationship between hybridisation in solution and hybridisation in DNA microarrays is nevertheless not clear yet.

Fish et al. (Nucleic Acids Research, 2007, 35 (21), 7197-7208) compared the microarray hybridization intensities of short duplex DNAs, designed for perfect match formation or containing tandem mismatches, with the thermodynamic stabilities ($\Delta G$ value) in solution, measured in DSC experiments. They observed significant discrepancies in the accuracy of the nearest-neighbour model in predicting thermodynamic stabilities of tandem mismatch duplexes.

Also, Wei et al. (Nucleic Acids Research, 2005, 33 (8), e78) describes a method to enhance the hybridization efficiency and to reduce hybridization time (or time to reach equilibrium) using microchannel hybridisation and chaotic mixing of droplets.

A better understanding of the molecular interactions may result in the possibility to turn microarrays into more precise tools.

**Summary of the invention**

It is an object of embodiments of the present invention to provide good methods for analysing or assessing hybridisation of targets.

It is an advantage of embodiments according to the present invention that methods and systems are provided for analysing hybridisation of targets, allowing identification and/or quantification of targets or providing relevant information regarding their hybridisation. It is an advantage of embodiments according to the present invention that methods and systems are provided for setting up hybridisation experiments for analysing samples.

It is an advantage of embodiments according to the present invention that these provide a better understanding of the physico-chemical properties involved in the free energy of the molecular interactions, and of the time scale and the dynamics of the hybridisation process. Embodiments according to the present invention allow a better characterisation of free energy, a better characterisation of the dynamics involved and allow taking this into account when performing hybridisation experiments.

It is an advantage of embodiments according to the present invention that more accurate quantification of interactions in microarrays, such as e.g. DNA microarrays, can be performed. It is an advantage of such embodiments that this may lead to a better understanding of the functioning of microarrays and of other techniques involving hybridization on a surface.

It is an advantage of embodiments according to the present invention that methods and systems are provided allowing hybridisation experiments in microarrays which are reliable, cheap and quick experiments. The methods and systems therefore are especially suited for diagnostics and personalised medicine, although embodiments of the present invention are not limited thereto.

The above objective is accomplished by a method and device according to the present invention.

The present invention relates to a method for analysing hybridisation between a target in a sample solution and a probe bound at a surface, the method comprising receiving detection results for hybridisation of the target with a plurality of different probes, the probes being selected so that a range of hybridisation detection intensity results for the hybridisation between the target and the probe is covered, and analysing the detection intensity results as function of hybridisation free energy, wherein the hybridization free energy for the hybridisatino between a target in solution and a probe bound at a surface is determined based on a model, the model being a model wherein a thermodynamic non-equilibrium state for the hybridization is taken into account and wherein analysing the detection intensity results as function of the hybridisation free energy comprises analysing the logarithm of the detection intensity results as function of hybridisation free energy for a range of hybridisation free energies and determining whether one linear relationship or a deviation therefrom, such as more linear relationships, can be distinguished between parts of the logarithm of the detection intensity results and the hybridisation free energy.

It is an advantage of embodiments according to the present invention that methods and systems are obtained allowing good analysis of the hybridisation results. It is an advantage of embodiments according to the present invention that reliable and accurate results can be obtained by analysis of hybridisation, e.g. in microarrays, allowing a wide range of hybridisation based microarray applications.

For said receiving detection intensity results or for said analysing reaching of thermodynamic equilibrium may be taken into consideration.

Said receiving detection intensity results taking into consideration reaching of thermodynamic equilibrium may comprise receiving detection intensity results for hybridisation of the target with a plurality of different probes obtained under hybridisation conditions wherein thermodynamic equilibrium has been reached.

The hybridisation conditions may comprise one or a combination of hybridisation time, probe length or temperature.

Analysing may comprise deriving that the hybridisation has not reached equilibrium when a deviation from the one linear relationship can be distinguished between parts of the logarithm of the detection intensity results and the hybridisation free energy.

More particularly, analysing may comprise deriving that the hybridisation has not reached equilibrium when a deviation from a linear relationship with a slope 1/RT can be distinguished. A deviation from the linear relationship may be a deviation over 5%, more preferably over 10%, still more preferably over 25%, even more preferably over 33%.

The method may comprise determining the hybridisation free energy for the hybridisation between a target in solution and a probe bound at a surface based on a nearest-neighbour model. It is an advantage of embodiments according to the present invention that hybridisation free energy can be determined accurately for hybridisation between a target initially in solution and a probe bound to a surface, such as for example may occur in microarrays.

For a given target, a perfect match probe, and probes with up to two non-complementary elements may be provided, each to separate microarray spots for interaction with the target during the hybridisation. The non-complementary elements may have a minimal effect on the hybridisation free energy. Analysing the detection intensity results as function of the hybridisation free energy may comprise determining a set of free energy parameters of a nearest neighbour model for the hybridisation free energy based on said hybridisation microarray experiment. It is an advantage of embodiments according to the present invention that good methods and systems are provided for setting up a model for determination of hybridisation free energy between a target in a solution and a probe bound to a surface.

Analysing the detection intensity results as function of the hybridisation free energy may comprise determining whether measured detection intensity results correspond with thermodynamic equilibrium for the hybridisation. It is an advantage of embodiments according to the present invention that based on these results, experiments can be designed so as to be performed in a particular thermodynamic state. The latter may for example be performed by adjusting the hybridisation time, the temperature, the probe length used, etc. Receiving detection intensity results may comprise receiving detection intensity results for hybridisation between the plurality of different probes and the target, the probes being selected so that a range of hybridisation detection intensities for the hybridisation between each possible target of the set of known possible targets and the probe is covered. Analysing the detection intensity results as function of the hybridisation free energy may comprise analysing the detection intensity results as function of the hybridisation free energy for each of the possible targets of the set of known possible targets and deriving the presence or concentration of an actual target by detecting a predetermined relationship between the detection intensity results and the hybridisation free energy.

The actual target may be a minority target and the set of known possible targets may comprise, besides the minority target, also a main target differing from the minority target in one or two non-complementary element.

The actual target may be an unknown target differing from a main target in one or two non-complementary elements, and the set of known possible targets may comprise the main target and a set of targets differing from the main target in one or two non-complementary elements.

The method may be for use in detecting single nucleotide polymorphisms.

Hybridisation may be performed in a microarray.

The detection intensity results for hybridisation may be induced by emission of a label associated with a hybrid formed by binding of the target and the probe.

The present invention also relates to a system for analysing hybridisation between a target in a sample solution and a probe bound at a surface, the system comprising a receiving means adapted for receiving detection intensity results for hybridisation of the target with a plurality of different probes, the probes being selected so that a range of detection intensities for the hybridisation between the target and the probe is covered, and an analysing means adapted for analysing the detection intensity results as function of the hybridisation free energy, wherein the analysing means is adapted for determining the hybridization free energy for the hybridisation between a target in solution and a probe bound at a surface based on a model, the model being a model wherein a thermodynamic nonequilibrium state for the hybridization is taken into account and wherein the analyzing means is adapted for analysing the logarithm of the detection intensity results as function of hybridisation free energy for a range of hybridisation free energies and for determining whether one linear relationship or a deviation therefrom, such as more linear relationships can be distinguished between parts of the logarithm of the detection intensity results and the hybridisation free energy.

The present invention also relates to a method for determining hybridisation free energy for the hybridisation of a target initially in solution and a probe bound to a surface, the method comprising performing a hybridisation microarray experiment using a plurality of microarray spots, wherein for a given target a perfect match probe, and probes with up to two non-complementary elements are provided to separate microarray spots for interaction with the target during the hybridisation microarray experiment, determining a set of parameters of a nearest neighbour model for the hybridisation free energy based on said hybridisation microarray experiment and applying the nearest neighbour model for determination of the hybridisation free energy.

The present invention further relates to a method for performing hybridisation, the method comprising performing hybridisation between a target initially in solution and a probe bound to a surface, and applying a dehybridisation step for removing cross-hybridised targets, wherein the dehybridisation step is performed during a dehybridisation time equal to a relaxation time of a hybridisation process between a target and a probe having one or two non-complementary elements, the relaxation time being determined using a method for analysing hybridisation as described above.

The present invention also relates to a controller adapted for controlling hybridisation experiments, the controller being adapted for performing hybridisation between a target initially in solution and a probe bound to a surface, and for applying a dehybridisation step for removing cross-hybridised targets, wherein the dehybridisation step is performed over a dehybridisation time equal to a relaxation time of a hybridisation process between a target and a probe having one non-complementary element, the relaxation time being determined using a method for analysing hybridisation as described above.

The present invention furthermore relates to a method for analysing hybridisation, the method comprising receiving detection intensity results for hybridisation between a target initially in solution and at least one probe bound to a surface, and analysing the detection intensity result as function of the hybridisation free energy, the method taking into consideration reaching of thermodynamic equilibrium determined using a method for analysing hybridisation as described above.

The present invention also relates to a hybridisation kit for hybridisation measurements for identifying an actual target out of a set of known possible targets, the hybridisation kit comprising a microarray having a plurality of microarray spots each of them comprising a probe, the plurality of different probes being selected so that the corresponding hybridisation covers a range of hybridisation detection intensities for the hybridisation of each possible target of the set of known possible targets.

The present invention furthermore relates to the use of a method for analysing hybridisation for designing a hybridisation kit.

Furthermore, the present invention relates to a computer program product for performing, when executed on a computing device, a method for analysing hybridisation as described above. It also encompasses a machine readable data storage device storing such a computer program product and transmission thereof over a local or wide area network.

Particular and preferred aspects of the invention are set out in the accompanying independent and dependent claims. Features from the dependent claims may be combined with features of the independent claims and with features of other dependent claims as appropriate and not merely as explicitly set out in the claims. These and other aspects of the invention will be apparent from and elucidated with reference to the embodiment(s) described hereinafter.

**Brief description of the drawings**

FIG. 1 illustrates a graph expressing the measured intensities in a hybridisation experiment using a plurality of different probes bound to a surface and a target as function of the hybridisation free energy whereby thermodynamic equilibrium has not been reached for all probe spots, as can be used in an embodiment according to the present invention.

FIG. 2 illustrates a graph as in FIG. 1, whereby thermodynamic equilibrium has been reached for all probe spots, as can be used in an embodiment according to the present invention.

FIG. 3A and FIG. 3B shows an example of a graph of detection intensity results as function of the hybridisation free energy for detection of a target from a known set of targets, whereby FIG. 3A illustrates the results when the supposed target used for the graph is the actual target that was in the sample, whereas FIG. 3B illustrates the results when the supposed target used for the graph is not the actual target that was in the sample, as can be obtained in an embodiment according to the present invention.

FIG. 4 illustrates graphs for analysis in a method for detecting a minority target differing a single nucleotide from a main target, as can be obtained in an embodiment according to the present invention.

FIG. 5 shows a flow chart for an exemplary method for performing hybridisation according to an embodiment of the present invention.

FIG. 6 indicates correlation plots for the total intensities (a) and the median intensities (b) in replicated hybridisation experiments of a first particular example illustrating features of embodiments according to the present invention.

FIG. 7 illustrates a plot of intensities (a) as function of $-\Delta\Delta G_{sol}$ for different concentrations and a plot of predicted behaviour (b) based on the Langmuir model for a first particular example, illustrating features of embodiments according to the present invention.

FIG. 8 shows ratios of intensities and perfect match intensities as function of $-\Delta\Delta G_{\mu,array}$ for a first particular example, illustrating features of embodiments according to the present invention.

FIG. 9 shows plots of $I/I^*_{pm}$ as function of the nearest neighbour fitted $-\Delta\Delta G_{\mu,array}$ for a first particular example, illustrating features of embodiments according to the present invention.

FIG. 10 shows a comparison of data in tables 4 and 5 between $\Delta\Delta G_{sol}$ and $\Delta\Delta G_{\mu,array}$ for a first particular example, illustrating features of embodiments according to the present invention.

FIG. 11 shows a plot of the intensity divided by concentration as function of $\Delta\Delta G$ for particular experiments of a second particular example with a different target concentration, illustrating features of embodiments according to the present invention.

FIG. 12 illustrates the three state model for hybridisation in DNA microarrays, as can be used in embodiments according to the present invention.

FIG. 13 illustrates a numerical solution of a fraction of hybridized probes for the three state model for a second particular example of hybridisation, illustrating features of embodiments according to the present invention.

FIG. 14 shows a plot of the intensity as function of $\Delta\Delta G$ for particular experiments of a second particular example at different hybridisation times, illustrating features of embodiments according to the present invention.

FIG. 15 shows a plot as shown in FIG. 14 for hybridisation with a shorter target sequence, illustrating features of embodiments according to the present invention.

Table 1 illustrates the oligos used as target in the four different hybridisation experiments of a first particular example illustrating features of embodiments according to the present invention.

Table 2 illustrates the design of the probe set used in a first particular example illustrating features of embodiments according to the present invention.

Table 3 illustrates the target conditions per microarray used in experiments of the first particular example illustrating features of embodiments according to the present invention.

Table 4 illustrates free energy differences parameters obtained from fitting microarray data to an equation expressing the logarithmic ratios of the intensities with the perfect match intensities for the first particular example, illustrating features of embodiments according to the present invention.

Table 5 illustrates data as in table 4, using the nearest neighbour parameters obtained from melting experiments in solution for the first particular example, illustrating features of embodiments according to the present invention.

Table 6 illustrates targets and probe sequences used in a second particular example, illustrating features of embodiments according to the present invention.

The drawings are only schematic and are non-limiting. In the drawings, the size of some of the elements may be exaggerated and not drawn on scale for illustrative purposes. Any reference signs in the claims shall not be construed as limiting the scope. In the different drawings, the same reference signs refer to the same or analogous elements.

Detailed description of illustrative embodiments

Where in embodiments of the present invention the term "equilibrium" is used, reference is made to thermodynamic equilibrium describing a situation wherein a steady state is obtained such that the number of conventional target-probe bindings does not substantially change over time. The term "non-equilibrium" or "non-equilibrium effects" is used to refer to occurrence of a target-probe binding state that may change over time. Where in embodiments of the present invention the term "free energy" is used, reference is made to the Gibbs free energy ($\Delta G$), referring to the thermodynamic potential that measures the "useful" energy obtainable from an isothermal isobaric thermodynamic system change. Where in embodiments analysis is performed as function of hybridisation free energy, this includes analysis as function of $\Delta\Delta G$ being the free energy difference between a perfect matching hybridization and an hybridization where the probe sequences have one or more internal mismatches.

The term hybridisation used in embodiments according to the present invention refers to nucleic acid hybridisation. This refers to the process of establishing a non-covalent sequence-specific interaction between two or more complementary strands of nucleic acids into a single hybrid. The strands of nucleic acids that may bind to their complement can for example be oligonucleotides, DNA, RNA or PNA. Nucleotides form the basic components of the strands of nucleic acids. Hybridisation comprises binding of two perfectly complementary strands (in the Watson-Crick base-pairing senses), but also binding of non-perfect complementary strands. With a non-perfect complementary strand reference may be made to strands having a small number of non-complementary elements such as one, two or more non-complementary elements, preferably one or two non-complementary elements. In principle there is no limit to the number of non-complementary elements but the more non-complementary elements, the easier these are detectable and thus the less it is required to have dedicated methods for detecting these. In some applications the latter is un-wanted and referred to as cross-hybridisation. Non-perfect complementary strands can contain different types of non-complementary elements like e.g. mismatches or loops, or any local alteration of binding properties. Non-complementary elements thereby may have a small effect on the free energy. The current invention is not limited to a particular type, but for clarity the examples given further below deal with small number of mismatches. Such a small number of mismatches may e.g. include one, two or more nucleotides that are mismatched. Hybridisation may occur between strands that both are in solution, but in the present embodiments, the hybridisation envisaged is the interaction between a strand that initially is in a sample solution and a strand that is bound to a surface. Where in embodiments of the present invention reference is made to the term "probe" a substance is envisaged that allows detection or identification of another substance in a sample. The probe may for example be a strand of oligonucleotides, DNA, RNA or PNA (partially) complementary to the strand of interest in the sample solution, e.g. referred to as "actual target" or target. Object of interest that may be present in a sample solution and for which a check may be performed may be referred to as a "possible target". In some embodiments according to the present invention, reference is made to a minority target and a main target, the minority target being a target different from the main target, e.g. being slightly different, and being present in a concentration substantially smaller than the main target. In hybridisation experiments according to the present invention, detection of hybridisation typically may be performed by a marker associated with the formed hybrid, such as for example a radioactive marker or a fluorescence marker, embodiments of the present invention not being limited thereto. Examples of typical fluorescence markers that may be used are Cy3 or Cy5 which are dyes of the cyanine dye family, the invention not being limited thereto. Typically in hybridisation experiments, intensity of the radiation or fluorescence provided by the markers is detected and representative for the number of hybrids formed.

Examples and embodiments of the present invention may be illustrated using a particular platform for microarrays, being the Agilent platform from Palo Alto. Nevertheless, it is to be noticed that the concepts and features set forth in embodiments according to the present invention are not limited to this particular platform but can be applied mutatis mutandis to other platforms, such as for example the GeneChips platform from Affymetrix or CodeLink Bioarray platform from Amersham Biosciences. Whereas the particular fitting parameters for the model used may be platform dependent, the principles and features of the methods set out in embodiments of the present invention can be applied to these and other platforms. Furthermore, embodiments of the present invention are not limited to microarrays, but may be applied for different hybridization measurements.

In a first aspect of the present invention, a method and system is described for analysing hybridisation between a target in a sample solution and a probe bound at a surface. The system and method may be especially suitable for analysing hybridisation in a microarray, although the invention is not limited thereto.

Embodiments of the present invention may also be applied for or assist in purification applications, applications for selection of sequences out of a mixture, diagnostics, applications for detecting small mutations, applications for detecting viruses, etc.

According to embodiments of the present invention, the method comprises receiving detection intensity results for hybridisation of the target with a plurality of probes, the probes being selected so that a range of hybridisation detection intensities for the hybridisation between the target and the probe is covered. The plurality of probes, and hybridisation may be tested or performed for each probe type in a single spot, e.g. in a microarray spot. Detection intensity results may be obtained based on label emission from a label associated with a hybride formed during hybridisation. The range of hybridisation detection intensities implies that a range of hybridisation free energies is covered. Receiving detection intensity results may comprise obtaining detection intensity results by actually measuring the intensity, thus including the detection or measurement step, or it may comprise receiving data via an input port for processing. By way of illustration, one example for obtaining detection results may encompass providing a plurality of different probes applied to different spots in a microarray. Each probe type thereby is bound to a surface, e.g. a microarray spot surface. Obtaining detection results furthermore may encompass providing a sample solution comprising the target to the surface to which the probes are linked and allowing the hybridisation process to take place. Such hybridisation may be platform dependent but may typically acquire at least 15 hours. As will be shown and discussed below, the hybridisation time used before measurement of the hybridisation, or factors related thereto such as the length of the probes or the temperature, may play an important role in obtaining accurate results. With the formation of hybrids, typically a marker may be associated. Such markers may for example allow optical detection of the hybridisation, although the invention is not limited thereto. Detection results thus may encompass emission intensities of markers associated with hybrids formed during hybridisation.

As indicated above, in thermodynamic equilibrium the hybridisation between a target initially in the sample solution and a probe bound to a surface is characterised by the hybridisation free energy ($\Delta$G). This hybridisation free energy $\Delta$G expresses the free energy difference between the bound probe-target state (double stranded) and its unbound state. A method for determining such hybridisation free energy ($\Delta$G) values in the case of hybridisation between a target initially in solution and a probe bound to a surface will be discussed below. The plurality of different probes being selected so that a range of hybridisation detection intensity results for the hybridisation between the target and the probe may comprise a probe being the perfect complement of the target one wants to detect and probes that are not perfectly complementary, for example having for example one or two nucleotides being mismatched to form the perfect complement of the target, showing loops, etc.. Depending on the application envisaged, the plurality of different probes may comprise such probes for a single target envisaged, for a main target and a minority target, for a set of targets of which one target is to be selected, etc.

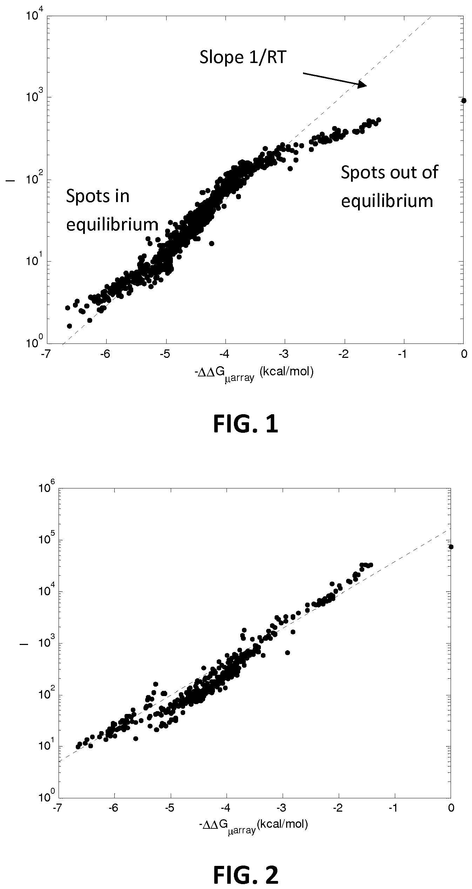

The method furthermore comprises analysing the measured or detected intensity as function of the hybridisation free energy. One exemplary way of analysing the measured or detected intensity as function of the hybridisation free energy is discussed with reference to FIG. 1, although embodiments of the present invention are not limited thereto.

Using the plurality of different probes as described above, the hybridisation experiment results in an intensity value per probe type. Evaluating the measured intensity as function of the hybridisation free energy, for example as function of the difference of hybridisation free energy with respect to the perfect match free energy for a possible target, allows for providing additional information regarding the hybridisation process itself and the hybrid, target and/or probe. As will be discussed later, such analysis may take into consideration reaching a thermodynamic equilibrium, for example, the analysis may take into consideration that the hybridisation is not in equilibrium yet. The analysis comprises an analysis of the logarithm of the detection intensity results as function hybridisation free energy for a range of hybridisation free energies. The analysis comprises determining whether one linear relationship or a deviation therefrom, such as more linear relationships can be distinguished between parts of the logarithm of the detection intensity results and the hybridisation free energy. A deviation from a linear relationship may be a deviation of more than 5%, e.g. more than 10%, e.g. more than 25%, e.g. more than 33%. The analysis may comprise determining whether a linear relationship with slope 1/RT can be distinguished. From this analysis different conclusions may be drawn, such as for example the presence of a target, the presence of a minority target, identification of one target out of a set of targets, identification that equilibrium has been reached or not, etc.

FIG. 1 illustrates the logarithm of the measured intensities as function of the difference of hybridisation free energy with respect to the perfect match free energy for a possible target. As shown above in FIG.1 some intensity measurements correspond with spots where the hybridisation is in thermodynamic equilibrium, while others are not. Furthermore, as illustrated in the applications, features such as equilibrium or not, presence of a target, concentration of target, identification of mutations, etc. may be derived in the analysis, as further illustrated under the different applications discussed below. As will be described later for a plurality of applications, the analysis also may comprise considering the above analysis for a plurality of targets and selecting the actual target based thereon.

According to embodiments of the present invention, receiving detection results and/or analysing the results may take into consideration reaching of thermodynamic equilibrium. In other words, receiving detection intensity results and/or analysing them may take into consideration the fact that no thermodynamic equilibrium state has been reached already. In some embodiments according to the present invention, the step of receiving detection results may take into consideration a thermodynamic non-equilibrium state of the hybridisation. The latter may for example encompass adapting the hybridisation conditions such that thermodynamic equilibrium or thermodynamic non-equilibrium can be obtained. Such hybridisation conditions may comprise for example the temperature at which hybridisation is performed, the length of the probes used, the hybridisation time used, etc. In some embodiments according to the present invention, the step of analysing takes into consideration a thermodynamic non-equilibrium state for the hybridisation. The latter may be performed by discarding a certain number of intensity results obtained, fitting predetermined correlations to part of the data obtained. In some embodiments, it is performed by fitting different predetermined correlations to the detected intensity, one being representative for the equilibrium state and one being representative for the non-equilibrium state.

Without wanting to be bound by theory, the results obtained with methods and/or system embodiments according to the present invention could be explained by the occurrence of non-equilibrium effects. More particularly, it was surprisingly found that, although typically the assumption of thermodynamic equilibrium was made in hybridisation experiments, thermodynamic non-equilibrium effects play a significant role. Experiments show that upon increase of the hybridisation time, substantially over times that are conventionally used in hybridisation experiments, more probes reach equilibrium. The experimental results for non-equilibrium effects could be explained by determination of the hybridisation free energy ($\Delta G$) which could be determined using a nearest neighbour model for the hybridisation. The nearest neighbour model for hybridisation between a target and a probe bound to a surface could be applied and model parameters could be determined due to a particular experimental design and dedicated custom microarrays, an example thereof being described further below. By extending the hybridisation theory, from the two-state model mentioned above to a three state model, the form of the experimentally determined non-equilibrium effects could be explained. As shown in FIG. 1, expressing the logarithm of the measured intensity I in the hybridisation experiments as function of the hybridisation free energy ($\Delta G$), only a partial confirmation of the linear relationship between log(I) and the hybridisation free energy ($\Delta G$) having a slope 1/RT could be obtained. It can be seen that there is also a deviating regime with an approximate linear behaviour between log(I) and the hybridisation free energy ($\Delta G$), at a different, i.e. smaller, slope.

It is to be noticed that, whereas embodiments of the invention mainly focus on systems and methods for analysing hybridisation or setting up hybridisation experiments, in one aspect embodiments of the present invention also relate to a method and system for determining hybridisation free energy for the hybridisation of a target initially in solution and a probe bound to a surface or for setting up a nearest neighbour model for the hybridisation free energy between a target initially in sample solution and a probe bound to a surface. The method thereby comprises performing a hybridisation microarray experiment using a plurality of microarray spots, wherein for a given target a perfect match probe and probes with up to two non-complementary elements such as two nucleotide mismatches are provided, e.g. by printing, on separate microarray spots for interaction with the target during the hybridisation microarray experiment. The method also comprises determining a set of parameters for a nearest neighbour model for the hybridisation free energy based on the hybridisation microarray experiment. Optionally the method may be directly applied for determination of the hybridisation free energy.

Turning back to the non-equilibrium conditions for hybridisation, it is to be noticed that for the applications, and their interpretation, it is advantageous to know in which regime one is measuring, since the regime influences the effect on intensity due to an additional non-complementary element, e.g. mismatch, in the target-probe duplex. If for example, one wants a probe that is as specific as possible to the perfect match target, in order to avoid that non-perfect matching targets hybridize the probe i.e. in order to avoid cross-hybridisation, it is important that the measurements are performed in the regime showing a relation between the logarithmic detection intensity and the hybridisation free energy with the highest slope, as this is the regime with the highest specificity.

In one embodiment a method is described for performing a hybridisation measurement especially suitable for identifying an actual target out of a set of known possible targets. The method comprises receiving detection intensity results for hybridisation of the actual but unknown target with a plurality of different probes, the probes being selected so that a range of hybridisation detection intensities for the hybridisation between each possible target of the set of known possible targets and the probe is covered. The latter directly implies that a range of free energies is covered. The method also comprises analysing the detection intensity results as function of the hybridisation free energy for each of the possible targets of the set of known possible targets and deriving the presence or concentration of the actual target by detecting a predetermined relationship between the detection intensity results and the hybridisation free energy. It is an advantage if reaching of equilibrium is taken into account, although the embodiment is not limited thereto. In a further application the actual target is a known minority target which is present together with a known main target which differs slightly from the minority target e.g. in one or two non-complementary elements such as by one or two nucleotide mismatches, and the application allows detection of presence of the minority target and a quantification of the relative concentration. In another application, the actual target is an unknown target differing slightly from a main target, e.g. in one or two non-complementary elements such as by one or two nucleotide mismatches, and the set of known possible targets comprises the main target and a set of targets differing from the main target in one or two nucleotides, and the application allows identification of the actual target. Further details and advantages are described in the exemplary applications as provided below.

Whereas embodiments according to this first aspect have mainly been described as methods, also systems are encompassed.

Systems according to the present aspects typically comprise a receiving means, e.g. an input port but alternatively also a hybridisation measurement setup, for receiving the detection intensity data as described above and a processing means or processor for analysing the results as described above. The system furthermore may comprise additional components for performing additional functionalities as described in the method embodiments described in the present application.

In one aspect, the present invention also relates to a method for performing hybridisation, wherein first a hybridisation step is performed between a target initially in the sample solution and a probe bound to a surface and thereafter a dehybridisation step is performed for removing cross-hybridized targets. The dehybridisation step is performed over a dehybridisation time substantially equal to the relaxation time of a hybridisation process between a target and a surface-bound probe having the unwanted non-complementary element, e.g. mismatch. The latter allows removal of such hybrids having the non-complementary element, while keeping the hybrids with perfect match. The relaxation time may advantageously be determined using a method as described above, applied to a target and a probe having the non-complementary element. An example of such a method for performing hybridisation is given below by way of an exemplary application. The present aspect also relates to a controller for controlling a hybridisation setup used for hybridisation experiments and thus for controlling hybridisation experiments as described above. The controller thus may be adapted for performing a hybridisation step and for performing a dehybridisation step for removing cross-hybridised targets, whereby the dehybridisation time advantageously is determined using a method for analysing hybridisation as described above. The controller may comprise a processor for performing an algorithm implementing the steps of a method for performing hybridisation as described above.

In another embodiment, the present invention relates to a method for analysing hybridisation comprising receiving detection intensity results for hybridisation between a target initially in solution and at least one probe bound to a surface, and analysing the detection intensity results as function of the hybridisation free energy. The method thereby takes into consideration the reaching of thermodynamic equilibrium which advantageously may be determined using a method for analysing hybridisation as described above.

In still another aspect of the present invention, a hybridisation kit for hybridisation measurements for identifying an actual target out of a set of known possible targets is described. The hybridisation kit comprises a microarray having a plurality of microarray spots each of them comprising a probe so that the plurality of different probes covers a range of detection intensities and corresponding therewith a range of hybridisation free energies for the hybridisation between each possible target of the set of known possible targets and the probes. The hybridisation kit may be especially suitable for performing applications as described in the present invention, although the invention is not limited thereto. The different probes may differ from each other or from one sub-set of the plurality of different probes only slightly, being by one or two non-complementary elements such as mismatches having a small influence on the hybridisation free energy.

In yet another aspect, the methods for analysing hybridisation may be used for designing a hybridisation kit, e.g. a kit as described above, although not limited thereto. The methods may in one example be used for determining whether for a given length of the probes equilibrium would be obtained and therefore may be used in the design of a hybridisation kit for determining a length of the probes used. In another example, the methods may be used for determining whether a sufficient range of detection intensity and corresponding therewith a sufficient range of hybridisation free energy is covered by a predetermined set of probes, and if required, the set of probes may be adjusted in view of this. Based on a the above methods for analysing hybridisation, a number of design guidelines may be derived.

The method for analysing hybridisation as described above thus also may be used as a method for calibrating or setting up a hybridisation experiment, used as method for calibrating a nearest neighbour model as will be described below, as well as to analyse the hybridisation experiments performed.

By way of illustration, the present invention not being limited thereto, a number of examples of applications are shown below that can be performed with or make use of embodiments of the present invention.

A first exemplary application relates to a system or method for determining whether a hybridisation experiment is in equilibrium or not. The method comprises performing a hybridisation experiment using a plurality of different probes, whereby the different probes are selected to comprise a perfect matching probe for the target and probes containing non-complementary elements, e.g. mismatches, with the target in such a way that a sufficient range of intensities and corresponding therewith ΔG is covered. The hybridisation experiment then results in an intensity value per probe. Evaluating the measured intensity as function of the hybridisation free energy, for example as function of the difference of hybridisation free energy with respect to the perfect match free energy for a possible target, allows for determining whether a spot has reached equilibrium or not. Spots that fulfil a predetermined relation between the logarithmic intensity measured and the hybridisation free energy, e.g. that fall on a line with slope 1/RT, are in equilibrium, spots deviating therefrom are not in equilibrium. As shown above in FIG.1 some spots may be in thermodynamic equilibrium, while others are not. The intensities corresponding to spots not in equilibrium do not fulfil a predetermined relationship between the logarithmic intensity and hybridisation free energy. In FIG. 1, only the spots on the bottom line in the graph correspond with spots that are in equilibrium. FIG. 2 illustrates an experiment whereby thermodynamic equilibrium is reached for all spots. The example shown in FIG. 2 is based on an example wherein the targets are shorter than in FIG. 1 (25 nucleotides instead of 30 nucleotides), allowing for quicker dynamics and consequently to an earlier reached equilibrium state.

A second exemplary application relates to a system or method for increasing specificity of hybridization experiments. The specificity is increased by minimising cross-hybridisation. The latter is obtained by, after a hybridisation experiment has reached equilibrium, applying a dehybridisation step for removing cross-hybridized targets. The dehybridisation step thereby is characterised by a dehybridisation time set equal to the relaxation time of the hybridization process for a target-probe complex having a non-complementary element, being a mismatch in the present example, as can be determined using a method according to an embodiment of the present invention. It may be set equal to the relaxation time of the target-probe complex having a non-complementary element having the smallest effect on the hybridisation energy (with reference to a perfect match) of all unwanted non-complementary elements. The relaxation time of target-probe complexes having at least one non-complementary element differs from, typically is shorter than, the dehybridisation time of a target-probe having no non-complementary element, as the hybridisation of the target-probe complex containing a non-complementary element has a lower free energy. To determine the optimal dehibridization time analysis is made of the graphs of log I as function of ΔG and selection of the dehibridisation time is performed for conditions wherein the ratio of the intensity corresponding with probes having perfect match to intensity corresponding with probes having the non-complementary element is maximal. The de-hybridisation may for example be performed by washing out the sample so that the target molecules are removed from the solution. The washing may be done with pure water or with water solution containing appropriated solvents. By applying the de-hybridisation step, most of the cross-hybridised targets will be removed, while most of the perfect matching targets will remain on the probe-spot. Such a system or method may be applied for microarray applications, although the invention is not limited thereto. The technique also can be applied for purification, for selection of sequences out of a mixture for example as a preliminary step for sequencing experiments, etc.

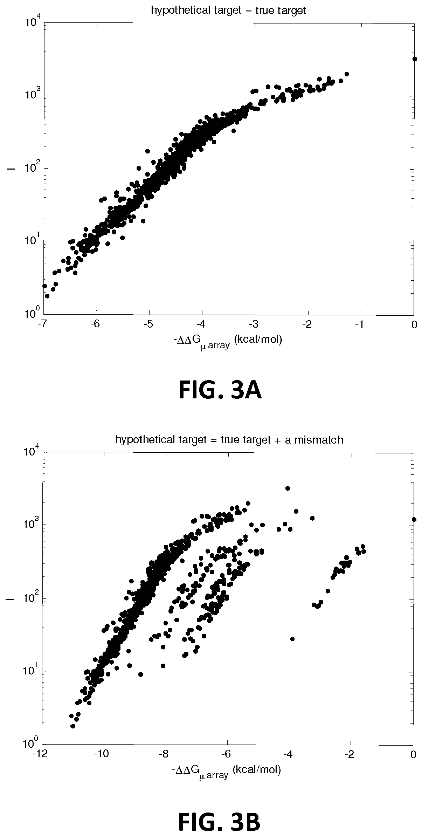

A third exemplary application relates to a system and/or method for identifying a target out of a set of known targets, i.e. one wants to know which of a known set of targets is in the solution. Whereas this problem could be solved by sequencing, the problem also could be solved by a hybridisation experiment. The system and/or method comprises performing a hybridisation experiment using a plurality of different probes, whereby the probes are selected to comprise, for each target of the set of known targets, a perfect matching probe for the target and probes containing non complementary elements with the target in such a way that a sufficient range of detection intensity and consequently of ΔG is covered. The hybridisation may be performed in a micro array experiment, although embodiments of the present invention are not limited thereto. The hybridisation experiment then results in an intensity value per probe. For each possible target of the known set of targets, the measured intensity for the plurality of different probes is evaluated as function of the hybridisation free energy of the possible target, e.g. as function of the difference between hybridisation free energy with respect to the perfect match free energy for the possible target. Based on this evaluation, identification of the actual target in the solution can be performed, as only in the case of the actual target, a predetermined correlation is detectable in the evaluation. By way of illustration, an example of such an evaluation is shown in FIG. 3A and FIG. 3B. The measured intensity is set out as function of the difference of hybridisation free energy with respect to the perfect match free energy for a possible target. The x-axis of the figure is dependent on the possible target selected, as the property on the axis is function of the free energy between the probe and the possible target, i.e. function of ΔG(probe_i, possible target). FIG. 3A illustrates the obtained result for a possible target corresponding with the actual target in the solution, resulting in a single curve or stated differently collapsing of the data points into a single curve, whereas FIG. 3B illustrates the obtained result for a possible target not corresponding with the actual target in the solution. In the latter case, no single curve is obtained. The actual target can thus be identified as the possible target for which the evaluation results in a predetermined relationship between intensity measured during the hybridisation experiments and the hybridisation free energy for the possible target ΔG(probe_i, possible target).

A fourth exemplary application relates to a system and/or method for detecting presence and/or quantifying the presence of a minority target in a solution comprising a main target being very similar to the minority target. With very similar there may be meant that there is only one or two non-complementary element, such as one or two nucleotide difference. The method and system according to embodiments of the present invention allows deriving the presence of a minority target and/or deriving an estimate of the proportion in which the minority target is present. It is an advantage of embodiments according to the present invention that the system and method for detection of minority targets in a sample solution that differ only slightly from a main target present in the solution is sensitive. It is an advantage of embodiments according to the present invention that a detection limit for minority targets can be obtained that is substantially better than 20%, as is for example the obtainable detection limit of Sanger sequencing techniques. It is an advantage of embodiments according to the present invention that detection may be performed based on hybridisation experiments. The system and/or method comprises performing a hybridisation experiment using a plurality of different probes, whereby the probes are selected to comprise, for both the main target and the minority target, a perfect matching probe for the target and probes containing non complementary elements with the target in such a way that a sufficient range of detection intensities and consequently of hybridisation free energy ΔG is covered.

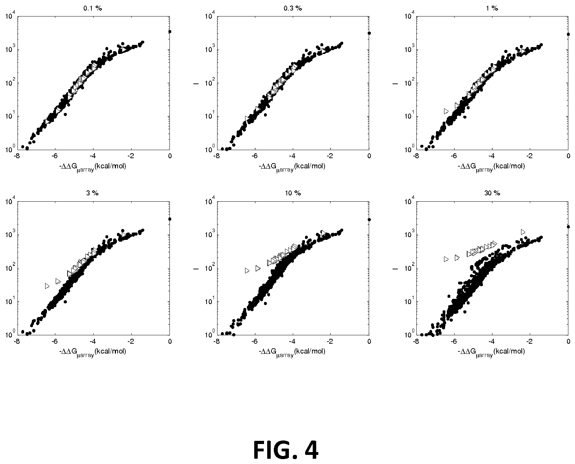

The hybridisation may be performed in a micro array experiment, although the invention is not limited thereto. The hybridisation is performed resulting in an intensity value for each probe. For the main target, the measured intensity for the plurality of different probes is then evaluated as function of the hybridisation free energy of the possible target, e.g. as function of the difference between hybridisation free energy with respect to the perfect match free energy for the possible target. Based on this evaluation, the presence of the minority target can be derived : if a single, predetermined correlation is derived, i.e. if the data collapse in a single curve, the minority target is not present within detection limit. If there is a deviation from a single curve or predetermined correlation, the minority target is present, and the proportion can be estimated. The latter may for example be performed, based on previously performed calibration measurements, or by theoretical calculation based on an extended equation correlating the logarithm of the measured intensity with the free energy ΔG. By way of illustration, a particular test experiment is shown in FIG. 4, illustrating the possibilities for detecting a minority target that differs a single nucleotide from a main target using a method as described above. Experiments were performed with samples containing a known concentration of minority target, varying over the different plots from 0.1% to 30%. In FIG. 4, the spots that are most sensitive to the presence of the minority target are indicated as triangles. It can be seen that from 1% à 3% onwards, in the present example, detection of the minority target is possible. Such a detection limit is substantially better than what is conventionally obtained using Sanger sequencing. The experiments can also be further optimised by taking into account the non-equilibrium effect occurring in hybridisation experiments, as described above. The latter typically results in that only the lowest spots indicated by triangles are in equilibrium and therefore are most sensitive. Quantification therefore preferably is based on these lower spots.

A fifth exemplary application relates to detection of a minority target in a sample, similar as described in the previous application, but wherein the minority target is not known as such, but only differs from the main target by one or two non-complementary elements, in the present example being one or two nucleotides. The sequence of the minority target then can be derived by performing an experiment as set out above, whereby it is not a priori known which probes correspond with the minority target. Identification of the probes corresponding with those intensity values deviating from the predetermined correlation for intensity as function of the free energy may allow for identification of the sequence of the minority target. It is an advantage of embodiments according to the present invention that such techniques can advantageously be applied for searching single nucleotide polymorphisms. It is an advantage of embodiments according to the present invention that alternative and complementary techniques with respect to purely statistical methods are provided for tackling searching of single nucleotide polymorphisms.

The different exemplary applications described above can be combined resulting in advantageous characterisation and detection methods.

While the invention has been illustrated and described in detail in the drawings and foregoing description, such illustration and description are to be considered illustrative or exemplary and not restrictive. The invention is not limited to the disclosed embodiments.

In one aspect, embodiments of the present invention also relate to computer-implemented methods for performing at least part of the methods for analyzing hybridization experiments or methods for setting up hybridization experiments.

Embodiments of the present invention also relate to corresponding computer program products. The methods may be implemented in a computing system. They may be implemented as software, as hardware or as a combination thereof. Such methods may be adapted for being performed on computer in an automated and/or automatical way. In case of implementation or partly implementation as software, such software may be adapted to run on suitable computer or computer platform, based on one or more processors. The software may be adapted for use with any suitable operating system such as for example a Windows operating system or Linux operating system. The computing means may comprise a processing means or processor for processing data. According to some embodiments, the processing means or processor may be adapted for performing analysis of one or more hybridization experiments according to any of the methods as described above. The processor therefore may be adapted for evaluating measured intensities as function of hybridization free energies and for determining therefrom an analysis result.

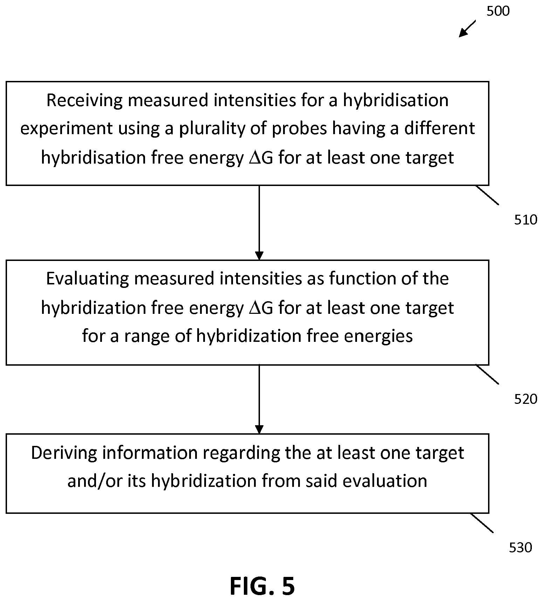

Performing such analysis may comprise for example determining whether hybridization with certain probes was measured in equilibrium, determining the presence of a known target, determining the presence and/or quantity of a minority target, identifying a minority target being present, etc. Besides a processor, the computing system furthermore may comprise a memory system including for example ROM or RAM, an output system such as for example a CD-rom or DVD drive or means for outputting information over a network. Conventional computer components such as for example a keyboard, display, pointing device, input and output ports, etc also may be included. Data transport may be provided based on data busses. The memory of the computing system may comprise a set of instructions, which, when implemented on the computing system, result in implementation of part or all of the standard steps of the methods as set out above and optionally of the optional steps as set out above. By way of illustration, the present invention not being limited thereto, an exemplary flow scheme of a computer implemented method is shown in FIG. 5. The computer implemented method 500 for analyzing hybridization may comprise receiving 510 measured intensities for a hybridization experiment using a plurality of different probes having a different hybridization free energy ΔG for at least one target. Such receiving may be receiving previously obtained data via an input means. Alternatively such receiving also may comprise obtaining the data directly through measurement during the hybridization experiment. The corresponding computing system therefore may comprise an input means for receiving input. The results also may be stored intermediately. The method further may comprise evaluating 520 measured intensities as function of the hybridization free energy for at least one target for a range of detection intensities and correspondingly a range of hybridization free energies. The range of detection intensities or hybridization free energies may be selected as function of the target to be characterized. Selection of such a range may for example be based on previously obtained results, or through trial and error. Evaluating may comprise determining a correlation between the measured intensities and the hybridization free energy or a property based thereon or being function thereof. Depending on the application, such an evaluation may be performed for one target, for a plurality of known targets that may be involved, etc. Such evaluation may be performed by an evaluation component of the processor. Based on the evaluation, e.g. on an established correlation between the intensities and the hybridization free energy, the computer-implemented method also may comprise deriving information regarding the at least one target and/or its hybridization. The latter may for example comprise determining the actual target present in the sample studied by hybridization, determining the presence and/or concentration of minority targets present in the sample studied by hybridization, detecting single nucleotide polymorphisms of targets in the sample studied, characterising the actual target present in the sample studied by hybridisation, determining whether the performed hybridisation experiment was in equilibrium for given probes, etc. Such information may be derived based on fitting of predetermined curves to the obtained correlation, evaluating deviation from predetermined curves, determining the slope of parts of curves, etc. The obtained results may be outputted through an output means such as for example a plotter, printer, display or as output data in electronic format.

The computing system and/or a corresponding computer implemented method may also be adapted for controlling the performance of the hybridization experiments themselves, although the invention is not limited thereby.

Further aspect of embodiments of the present invention encompass computer program products embodied in a carrier medium carrying machine readable code for execution on a computing device, the computer program products as such as well as the data carrier such as dvd or cd-rom or memory device. Aspects of embodiments furthermore encompass the transmitting of a computer program product over a network, such as for example a local network or a wide area network, as well as the transmission signals corresponding therewith.

By way of illustration, the present invention not being limited thereby, an example is given on how the free energy parameters of the nearest neighbour model could be fitted to results obtained for hybridisation in DNA microarrays. The example illustrates features and advantages of embodiments according to the present invention. Whereas for the present example theoretical considerations are taken into account, embodiments of the present invention are not limited thereby. For the present study several hybridization experiments were performed, each with a single oligonucleotide sequence (referred to as the target in this paper) in solution at different concentrations. Four different targets were used in the experiments, and their sequences are given in Table 1. The sequences contain a 30-mer hybridizing stretch followed by a 20-mer poly(A) spacer and a Cy3 label at the 3'-end of the sequence. Each target oligo was bought in duplicate in order to check the quality of the target synthesis. Reference will be made to the two duplicated oligos as a and b.

The sequences printed at the microarray surfaces and referred here as the probes were chosen to contain up to two mismatches, following the scheme shown in Table 2. Mismatches were inserted from nucleotides 6 to nucleotide 25 along the 30-mer sequences in order to avoid terminal regions. In the probes with two mismatches these were separated by at least 5 nt. Given the nucleotide of the target strand there are three different possible mismatching nucleotides and 20 available positions, hence in total 60 single mismatch sequences. A similar counting for double mismatches yields 945 different sequences (Table 2). The total number of probe sequences, including the perfect matching one, is 1006. For each experiment one target and one 8x15K custom Agilent slide was used. This slide consists of eight identical microarrays and each of these can contain up to more than 15 000 spots.

The 1006 probe sequences were spotted in the custom array 15 times: in 12 replicates a 30-mer poly(A) was added on the 3'-side (surface side), in order to asses the effect of a sequence spacer. Three replicates contained no poly(A) spacer. The eight microarrays of one slide have to be hybridized during the same experiment, but a different target solution can be used. In the experiments, the target concentrations ranged from 50 to 10000 pM according to the scheme given in Table 3. In Experiment 1 only target a was used, while in the Experiments 2, 3 and 4 both replicated targets (a and b) were used. Finally, in Experiments 1 and 2 a fragmentation of the target was performed before hybridization (see section on hybridization protocol for details). The four 30-mer target sequences were selected from fragments of human genes having a GC content ranging from 43 % to 50 %. A criterion for selecting the target sequences was the requirement that the probes constructed following the scheme in Table 2 would yield a roughly flat histogram of mismatch types, so that all mismatches are approximately equally present in the experiments.

For the experiments, the commercially available Agilent platform was used and a standard protocol with Agilent products was followed, as described subsequently. The target oligonucleotides were OliGold© from Eurogentec, Seraing, Belgium.

Hybridization mixtures contained one target oligonucleotide with a 3'-Cy3 endlabeling diluted in nuclease-free water to the final concentration together with 5 $\mu$l 10 x blocking agent and 25 $\mu$l 2 x GEx hybridization buffer HI-RPM. In Experiments 1 and 2 the addition of the hybridization buffer was proceeded by a fragmentation step, 1 $\mu$l fragmentation buffer was added followed by an incubation of 30 min at 60° C. This fragmentation buffer is customarily used in Agilent hybridization platforms and produces target sequences of reduced length in order to speed up the hybridization reaction. Too long sequences, as obtained from biological extracts, e.g. from reverse transcription of mRNA samples, have a reduced hybridization efficiency due to steric hindrance. By comparing experiments with and without fragmentation, it was found that the fragmentation step has little effect on the results. The hybridization mixture was centrifuged at 13 000 r.p.m. for 1 min and each microarray of the 8x15K custom Agilent slides was loaded with 40 $\mu$l.

The hybridization occurred in an Agilent oven at 65°C for 17 h with rotor setting 10 and the washing was performed according to the manufacturer's instructions. The arrays were scanned on an Agilent scanner (G2565BA) at 5 mm resolution, high and low laser intensity and further processed using Agilent Feature Extraction Software (GE1 v5 95 Feb07) that performs automatic gridding, intensity measurement, background subtraction and quality checks.

In the present example, use is made of the Langmuir model for describing the dynamics of hybridization by a rate equation for $\theta$, the fraction of hybridized probes from a spot as follows

$$\frac{d\theta}{dt} = ck_1(1-\theta) - k_{-1}\theta \qquad [3]$$

where c is the target concentration and $k_1$ and $k_{-1}$ are the attachment and detachment rates. The equilibrium value for $\theta$ can be obtained from the condition $d\theta_{eq}/dt=0$. Using the link between the rates and equilibrium constants, i.e. $k_1/k_{-1} = e^{-\Delta G/RT}$, with $\Delta G$ the hybridization free energy, R the gas constant and T the temperature one finds

$$\theta_{eq} = \frac{ce^{-\Delta G/RT}}{1+ce^{-\Delta G/RT}} \qquad [4]$$

which is the so-called Langmuir isotherm. To link this isotherm to the measured quantities one assumes that the fraction of hybridized probes is linearly related to the measured fluorescent intensity measured from a spot, which yields

$$I = \frac{Ace^{-\Delta G/RT}}{1+ce^{-\Delta G/RT}} \qquad [5]$$

Here I is the background-subtracted intensity, where the background subtraction, as explained above is done by Agilent Feature Extraction software. Where reference is made to the intensities, this are intensities that are background subtracted. A is a constant which is an overall scale factor.

Far from chemical saturation, i.e. when only a small fraction of surface sequences is hybridized (i.e. $c\,e^{-\Delta G/RT} <<1$) one can neglect the denominator in Equation [4] to get:

$$I \approx Ace^{-\Delta G/RT} \qquad [6]$$

In the nearest neighbour model, the hybridization free energy of perfect complementary strands is approximated as a sum of dinucleotide terms. For instance

$$\Delta G \begin{pmatrix} ATCCT \\ TAGGA \end{pmatrix} = \Delta G \begin{pmatrix} AT \\ TA \end{pmatrix} + \Delta G \begin{pmatrix} TC \\ AG \end{pmatrix} + \Delta G \begin{pmatrix} CC \\ GG \end{pmatrix} + \Delta G \begin{pmatrix} CT \\ GA \end{pmatrix} + \Delta G_{init} \qquad [7]$$

where $\Delta G_{init}$ is an initiation parameter. Since only differences of $\Delta G$ between a perfect matching hybridization and a hybridization with one or multiple mismatches [Equation [9]] are considered, this initiation parameter will not contribute and it is omitted further in this example. For DNA/DNA hybrids, symmetries reduce the number of independent parameters to 10. The nearest neighbour model can be extended to include single internal mismatches; as an example it is considered that the free energy of a stretch with an internal mismatch of CT type

$$\Delta G \begin{pmatrix} AT\underline{C}CT \\ TA\underline{T}GA \end{pmatrix} = \Delta G \begin{pmatrix} AT \\ TA \end{pmatrix} + \Delta G \begin{pmatrix} T\underline{C} \\ A\underline{T} \end{pmatrix} + \Delta G \begin{pmatrix} GT \\ C\underline{C} \end{pmatrix} + \Delta G \begin{pmatrix} CT \\ GA \end{pmatrix} \qquad [8]$$

The mismatching nucleotides are underlined and for notational reasons the mismatch is always put in the second part of the dinucleotide (which requires the use of symmetry like here in dinucleotide term three). There are 12 types of mismatches and 4 types of flanking nucleotide pairs, hence in total there are 48 mismatch parameters of dinucleotide type. There are several possible ways of extracting the 48 + 10 dinucleotide parameters from the experimental data.

One can either fit the full Langmuir isotherm [Equation [4]], or for experiments at sufficiently low concentrations one could consider the limiting case of Equation [6]. In addition, the parameters could be extracted either from an experiment at fixed concentration c, by comparing the intensities of different probe sequences, or from experiments at different concentrations by analyzing the intensities of identical probe sequences over a concentration range. Focus is put on the low concentration data and use of Equation [6] for the analysis at fixed c. Equation [6] contains the constant A which is an overall scale factor relating the hybridization probability to the actual measured fluorescence intensity. This quantity may fluctuate from experiment to experiment. For instance, the optical scanning influences A, as this is proportional to the laser intensity used. Also hybridizations in different slides might occur at slightly varying conditions and there can be small differences in the manifacturing of the slides. Focus will be put on relative intensities and relative free energies, i.e. for each microarray the perfect match of that microarray will be used as a point of reference.

The logarithmic ratios of the intensities with the perfect match intensity are denoted as

$$y_i = \ln I_i - \ln I_{PM} = -\frac{\Delta G - \Delta G_{PM}}{RT} = -\frac{\Delta \Delta G}{RT} \qquad [9]$$

for which the exact value of A is irrelevant and only the relative free energy differences $\Delta \Delta G$ (which is for each probe a positive number) are considered. In $\Delta \Delta G$ of a duplex, only dinucleotide parameters which are flanking a mismatch remain, the other parameters cancel out in the subtraction. For example from Equations [7] and [8] one gets

$$\Delta \Delta G \begin{pmatrix} AT\underline{C}CT \\ TA\underline{T}GA \end{pmatrix} = -\Delta G \begin{pmatrix} T\ \underline{C} \\ A\ \underline{T} \end{pmatrix} + \Delta G \begin{pmatrix} GT \\ C\underline{C} \end{pmatrix} - \Delta G \begin{pmatrix} TA \\ AT \end{pmatrix} - \Delta G \begin{pmatrix} AC \\ TG \end{pmatrix} \qquad [10]$$

In this equation the lower strand refers to the target sequence in solution, which is fixed. The upper strand is that of the probe sequence attached to the solid surface.

Hence, the $\Delta \Delta G$ of a duplex with one mismatch can be written as a sum of two mismatch dinucleotide parameters minus two matching dinucleotide parameters. As it is assumed that the nearest neighbor model is valid, the same reasoning can be applied to duplexes with two mismatches which results in a sum of four mismatch dinucleotide parameters minus four matching dinucleotide parameters. The model can now be written as

$$y_i = \sum_{\alpha=1}^{58} X_{i\alpha} \frac{\Delta G_\alpha}{RT} \qquad [11]$$

where a is the index running over the 58 possible dinucleotide parameters and X is a frequency matrix, whose elements $X_{i\alpha}$ are the number of times the dinucleotide parameter $\alpha$ enters in $\Delta \Delta G$ of probe sequence *i*. With a simple extension of matrices and vectors one can rewrite the problem as

$$\vec{y} = X\vec{\omega} \qquad\qquad [12]$$

Where it is defined that
$\omega_\alpha = \Delta G_\alpha / RT$. Having written the problem in Equation [12] as a linear one, one can now apply the standard approach to find the optimal values of the parameters. The procedure consists in minimizing $S = (\vec{y} - X\vec{\omega})^2$ which amounts to solving the following linear equation

$$X^T(\vec{y} - X\vec{\omega}) = 0 \qquad\qquad [13]$$

where $X^T$ is the transpose of X.

To obtain $\vec{\omega}$ from Equation [13] one has to invert the 58 x 58 matrix $X^TX$. In the case that $X^TX$ is not invertible one applies a singular value decomposition. In the present case the matrix is not invertible. Zero eigenvalues of the matrix $X^TX$ come from reparametrizations that leave the physically accessible parameters $\Delta\Delta G$ invariant.

The dinucleotide mismatch parameters are not uniquely determined, as these parameters are entering in the expression for the total $\Delta G$ in pairs [Equation (8)]. For instance, a reparametrization of the type:

$$\Delta G' \begin{pmatrix} x & \underline{C} \\ x' & \underline{T} \end{pmatrix} = \Delta G \begin{pmatrix} x & \underline{C} \\ x' & \underline{T} \end{pmatrix} + \varepsilon$$
$$\Delta G' \begin{pmatrix} y & \underline{T} \\ y' & \underline{c} \end{pmatrix} = \Delta G \begin{pmatrix} y & \underline{T} \\ y' & \underline{C} \end{pmatrix} - \varepsilon \qquad\qquad [14]$$

for every pair of complementary nucleotides x; x' and y; y' leaves the total $\Delta G$ invariant, as it can be verified directly from Equation [8]. Similar reparametrizations are possible for mismatches of type AG, AC and TG. Next to these there are three invariances of $\Delta\Delta G$ that involve a reparametrization of both mismatch and matching dinucleotide parameters. Hence one has at least seven zero eigenvalues in $X^TX$.

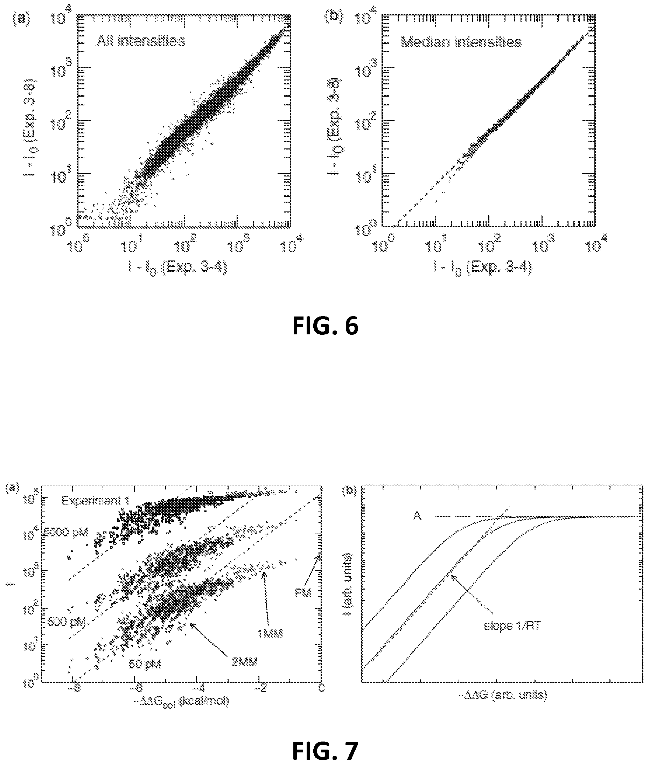

As a control of the reproducibility of the result, the intensities correlation between analogous spots in replicated experiments is considered. The replicated hybridizations were carried out on two microarrays of the same slide, with two identical but separately synthesized and labeled target oligos, at the same manually prepared concentration in solution Table 3. FIG. 6 is an example thereof. It shows correlation plots between two replicated hybridizations. Two plots are shown, one with the full 15K intensities FIG. 6(a) and one in which the median of the intensities of the 15 replicated spots are taken FIG. 6(b). In the former some data spreading is observed, which is greatly reduced when the median over 15 replicated spots is taken. Note that the experimental data do not align perfectly on the diagonal of the graph, this may be attributed to the manual preparation of the solutions or to differences in the oligos (synthesis or labeling). Data from different microarrays are aligned on a line of slope equal to one in the log-log plots of FIG. 6, which implies a linear relationship between the intensities. In general, replicates show a strong correlation between median intensities, which is an indication of a good reproducibility of the results. In this median the probes with and without poly(A) spacer are included. No significant difference was found in the intensities from spots with poly(A) and without poly(A) spacer. From this point on, the median intensity of 15 replicates is always used and simply referred to as the intensity of a probe, and because of the good reproducibility only the data produced by hybridizations with oligo synthesis a (Table 3) will be discussed .Next, the relation between the intensities and the corresponding $\Delta G_{sol}$ for hybridizations in solution with one or two mismatches are considered. In the case of two mismatches $\Delta G_{sol}$ was calculated as the sum of nearest neighbour parameters for individual mismatches, assuming that the presence of two mismatches does not involve additional terms in the free energy, i.e. they do not interact. In the experiment the minimal distance between two mismatches is 5 nt, which is considered sufficient, in first approximation to support the non-interaction assumption. In the calculation of $\Delta G$ from the tabulated values of $\Delta H$ and $\Delta S$ the temperature was set to the experimental value T=65°C.

FIG. 7(a) shows plots of the intensities versus $-\Delta\Delta G_{sol}$ as taken from the nearest neighbour model with the existing tabulated values for hybridization in solution. $\Delta\Delta G_{sol}$ is obtained by subtracting from all free energies that of the PM sequence, which is taken as a reference. As a consequence, for the PM intensities $\Delta\Delta G_{sol}=0$. Each plot in FIG. 7 contains 1006 data points obtained from the median value of the 15 replicated spots on each array.

As it is well-known from several studies of melting/ hybridization in aqueous solution, the hybridization free energy $\Delta G_{sol}$ depends on the buffer conditions, and in particular of the ionic strenght of the solution. Particularly studied was the effect of salt concentration (NaCl), which is usually assumed to be independent of sequence, but to be dependent on oligonucleotide length. Melting experiments in solution are consistent with the following dependence on Na ion

concentration

$$\Delta G_{sol} = \Delta G_{sol} \; (1M[Na^+]) - \alpha N ln[Na^+] \qquad\qquad [15]$$

Where $\Delta G_{sol} \; (1M[Na^+])$ is measured at 1M NaCl, N is the number of phosphates in the sequence and a is a constant.

Salt has mostly an effect on interactions with the negatively charged phosphate molecules. It is hence plausible to expect the same type of correction as Equation [15] also for sequences carrying mismatches. If that is the case, the salt dependence cancels out from $\Delta\Delta G_{sol}$, which is the quantity of interest. Therefore the value will be set at 1 M NaCl in $\Delta G_{sol}$.

FIG. 7(a) shows the data for Experiment 1 at three different concentrations, from bottom to top of 50, 500 and 5000 pM. When plotted as functions of $-\Delta\Delta G_{sol}$, the data points tend to cluster along single monotonic curves. This already suggests a fair degree of correlation between $\Delta G_{sol}$ and $\Delta G_{\mu array}$. The experiment at 5000pM shows a pronounced saturation of the intensities, as expected from the Langmuir model [Equation [4]]. Sufficiently far from saturation one expects a linear relationship between the logarithm of the intensity and $\Delta G$, as given by Equation [6]. FIG. 7 shows that the low concentration data at low intensities follow approximately a straight line with the slope 1/RT expected from equilibrium thermodynamics at T = 65°C, which is the experimental temperature.

However, the global behavior of the three concentrations is at odds with the Langmuir model, which predicts that intensity versus free energy plots for different concentrations should saturate at a common intensity value A, as indicated in FIG. 7(b). Although one may expect some variations on A from experiment to experiment, the data of FIG. 7(a) are hard to reconcile with the Langmuir model. It is concluded that the hybridization data deviate from the full Langmuir model of Equation [4], but they are in rather good agreement with its limiting low intensities behavior [Equation [6]]. In order to obtain estimates of the free energies $\Delta\Delta G_{\mu array}$ from microarray data, then Equation [6] will be used and restriction will be made to the lower concentration data.

To fit the 58 parameters of the nearest neighbor model the lowest concentration data are used, i.e. 50 pM. Hereto the algebraic procedure explained above is applied, which fits the logarithm of the ratios $I/I_{PM}$ and which assumes that the data can be described by Equation [9]. For low concentrations this assumption is expected to be correct for the lower intensities but not for the highest intensities, which deviate from the Langmuir isotherm as shown in FIG. 7. This poses a problem for the fitting procedure since it was designed with the perfect match intensity $I_{PM}$ as a reference [Equation [9]]. One may think to circumvent this problem by restricting the fit to low intensities, for instance only to probes with two mismatches and rewrite Equation [9] using as reference not $I_{PM}$, but for instance one of the intensities of a probe with two internal mismatches. This procedure turns out to be of little practical use for the purpose of estimating the free energy difference between perfect matching sequences and sequences with one or multiple mismatches and for which the PM reference value is necessary.

From the analysis of plots of intensity versus $-\Delta\Delta G_{sol}$ (FIG. 7), one finds that the PM intensity is systematically lower than that predicted by Equation [6], which is the straight line in FIG. 7(a). Hence, the relative intensities $I/I_{PM}$ of the probes that contain mismatches are systematically higher than those predicted by Equation [6].

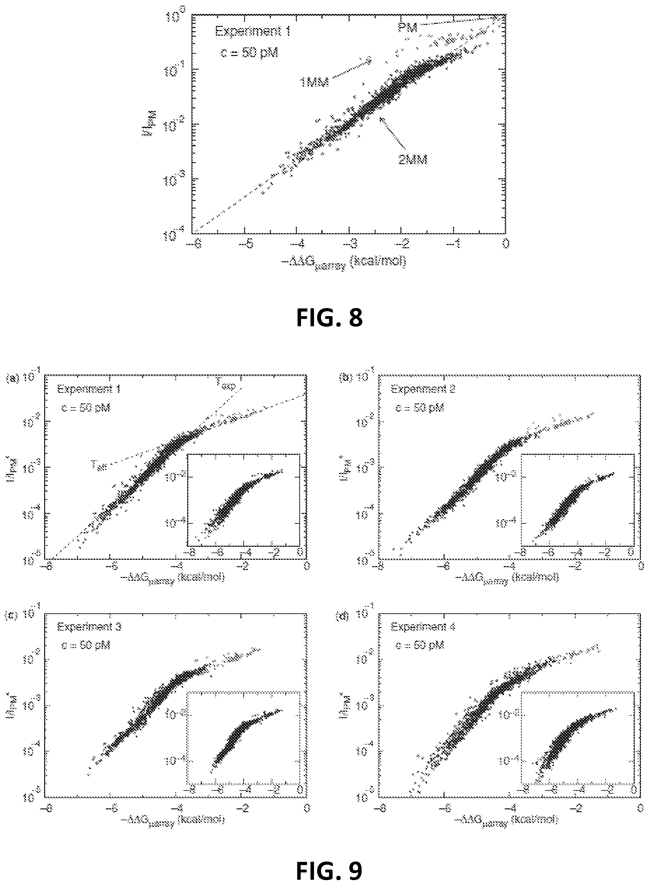

Consequently, a direct fit of the experimental data to Equation [9] underestimates the effect of a mismatch, which will result in free-energy penalties that are too small. The result of the fit is shown in FIG. 8. One can notice that the $\Delta\Delta G$ range is indeed smaller than the one from hybridization in solution (FIG. 7).

Moreover, the underestimation of $\Delta\Delta G$ is more severe for probes with two mismatches than for those with only one, since $\Delta\Delta G$ is a sum of contributions per mismatch. This produces a discontinuity of the curve from double to single mismatches. The appearance of this discontinuity is another evidence of the fact that Equation [6] is not valid in the full range of intensities.

In order to solve this problem, one would need to fit the data with a more general model $I(c,\Delta G)$ that incorporates the observed deviations from Equation [6]. Moreover, the choice of this model may considerably influence the fitted nearest neighbor parameters. A safer compromise is to start from the observation that Equation [6] is followed by the large majority of the low concentration data points in FIG. 7. Hence a fit to the low concentration limit of the Langmuir model seems reasonable. Unfortunately, one of the points deviating from Equation [6] is the PM intensity, which is used as reference measure. In order to calibrate the fit correctly one should reweight the reference PM intensity. The data therefore were fitted against Equation [9] using instead of the actual PM intensity as a reference, a rescaled value $I_{PM}^* = \alpha \, I_{PM}$, which is the value the PM intensity would have if the data would agree with Equation [6] in the whole intensity range. $\alpha$ is estimated from the crossing of the 50pM fitting line in FIG.7a with the $\Delta\Delta G=0$ axis. This estimate is $\alpha$=30.

The effects of a change in $\alpha$ on the fitting parameters will be discussed below.

FIG. 9(a-d) show the result of the fit to Equation [9], using $\alpha$ =30. In the main frames each experiment is fitted independently. In the insets, the free-energy parameters are obtained from a simultaneous fit of all 50pM experiments.

The latter data produce more accurate parameters, as they come from using four independent experiments (the four experiments at 50 pM, oligo synthesis a, in Table 3), hence the 58 parameters are obtained on sampling over 1006 x 4 data points. Both the free-energy range and the continuity of the curves in FIG. 9 are now as expected. The data show very little spreading in comparison with the curves in FIG. 7(a). A quantification of the spreading for a monotonic curve can be assessed by the Spearman's rank correlation coefficient, which for all four experiments is very close to 1. This is an indicator of the reliability of the nearest neighbor fitted parameters. The ratio of data points over tuning parameters is as large as 4024/58, which ought to yield a reliable fit. Moreover, although the data are fitted to a linear model, all four experiments show a clear deviation for the highest intensities.

This is an indication against overfitting, which would result in a fully linear curve with erroneous fitting parameters. Therefore, it is concluded that the deviations from the Langmuir isotherm observed in all four experiments is a robust feature of the system and that the resulting free-energy parameters are physically meaningful. It is also verified that the free-energy parameters obtained from the fit are quite stable whether one fits the whole set of experimental data, or whether the fit is restricted to the lowest intensity scales (e.g. $I/I^*_{PM} \leq 5 \times 10^{-3}$) where all data clearly follow Equation [6]. This is because the large majority of experimental points in FIG. 9 are located in the lowest intensity scales, anyhow. Hence, this additional data filtering has little effects on the parameters.

Table 4 shows the free-energy parameters $\Delta\Delta G_{\mu array}$ as obtained from the above fitting procedure. Because of the degeneracies mentioned above, the dinucleotide parameters are not uniquely determined. Triplet parameters are, however, unique, and these are given in the table. The $\Delta\Delta G$ for triplets are defined, for instance

$$\Delta\Delta G \left(\frac{A\underline{C}G}{TT\underline{C}}\right) = \Delta G \left(\frac{A\underline{C}G}{TT\underline{C}}\right) - \Delta G \left(\frac{AAG}{TTC}\right) \qquad [16]$$

where the upper strand is 5'-3' oriented. The lower strand is the invariant target sequence, the upper strand is the probe sequence. Hence the $\Delta\Delta G$ parameters are measured subtracting the reference perfect match probe. Note that because of this subtraction one has

$$\Delta\Delta G \left(\frac{A\underline{C}G}{TT\underline{C}}\right) \neq \Delta\Delta G \left(\frac{C\underline{T}T}{G\underline{C}A}\right) \qquad [17]$$

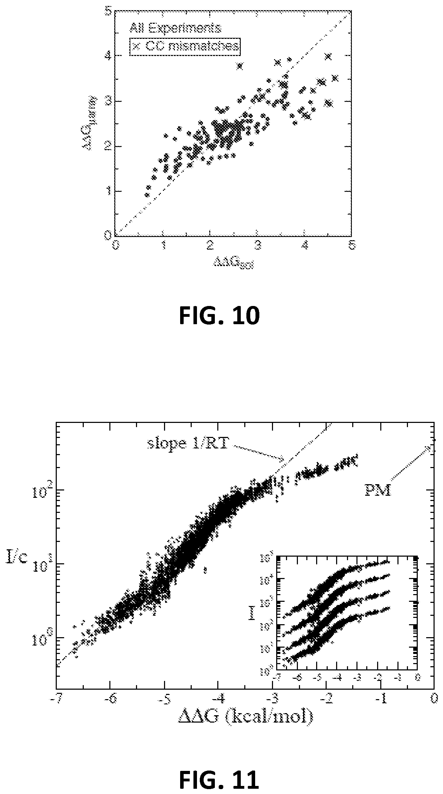

as the reference PM sequence is different in the two cases. Using standard linear regression tools, the error bar was estimated on the parameters of Table 4 to be equal to 0.2. In Table 5 the $\Delta\Delta G_{sol}$ for triplets following the same notation as in Table 4 are presented. As mentioned before the data in solution are at T = 65°C and 1M [Na$^+$]. FIG. 10 shows a plot of the two free energies $\Delta\Delta G_{\mu array}$ versus $\Delta\Delta G_{sol}$. A clear quantitative correlation between the two is observed. The Pearson correlation coefficient is 0.839. In comparing the two sets, it can be noted that the 16 mismatches of CC appear to be the most deviating in the two cases.

As discussed above, the fit was done with a re-scaled PM intensity, using a factor $\alpha$ =30. The analysis was repeated for other values of $\alpha$. Varying $\alpha$ causes a global shift of the data in Table 4 by an $\alpha$-dependent constant.

This shift does not affect the slope or correlation of the data in FIG.10. By using $\alpha$=50 a positive shift of 0.17 was found, while setting $\alpha$ =20 produces a shift of -0.14. These two values of $\alpha$ are our estimate of the largest range of variability for this parameter. In general, the procedure of re-weighting the PM intensity with $\alpha$ introduces a global error $\pm 0.2$ affecting all parameters in Table 4.

One of the advantages of the experimental setup chosen in this work is that one can obtain in principle all parameters in a single experiment, as all hybridization reactions with one or two mismatches occur in 'parallel' on a single array. However, a drawback is that in this setup one can determine only the free energy and not the contribution of enthalpy and entropy separately, which would allow to extend the parameters to other temperatures.

In the above example, focus was put on the determination of $\Delta\Delta G$ which is the free energy difference between a perfect matching hybridization and an hybridization where the probe sequences have one or more internal mismatches. Quantifying the effect of internal mismatches is important for a better understanding of cross-hybridization effect, which is the unintended binding of non-perfectly complementary sequences to a given probe. Moreover, this understanding could have some practical consequences for optimal probe design. An advantage of the parameter $\Delta\Delta G$ is that it is insensitive to the free-energy initiation parameter [Equation [7]] and the scaling factor A [Equation [4] and [6]] and that it is expected to be less sensitive to buffer conditions as ionic salt etc. The example, showing custom Agilent arrays shows that there is a strong correlation, also on the quantitative scale, between $\Delta\Delta G_{sol}$ and $\Delta\Delta G_{\mu array}$. This correlation is shown in FIG. 10 with explicit free-energy values given in Table 4 and 5. A fit of the interaction parameters from microarray data shows a much better agreement of the data with the thermodynamic models (compare FIG. 7 with FIG.

8). However, in the absence of dedicated experiments for the determination of interaction free energies on a DNA microarray, the results of this work suggest that one could use the corresponding hybridization free energies in solution as approximations for them.

As a correlation between $\Delta G_{sol}$ and $\Delta G_{\mu array}$ has by now been observed in several different microarray platforms, it is fair to expect that such a correlation is a general feature of microarrays.

It is interesting to remark that the deviation from the Langmuir model 'enhances' the cross-hybridization problem because there is a smaller effect on intensity for a given free energy penalty (smaller slope in FIG. 9). As an example, a mismatch with $\Delta\Delta G$ = 2.5 kcal/mol (a typical value from Table 4) corresponds to a $I/I_{PM}$ ratio of $\approx$ 0.02 in the regime governed by the Langmuir model, compared to $\approx$ 0.2 in the deviating regime.

This implies that in the deviating regime a significant fraction of the amount of target binding to a PM probe binds to a probe carrying one internal mismatch.

In a second example, hybridisation in DNA microarrays is discussed as also discussed in the first example. The second example illustrates the existence of slow relaxation phenomena for hybridisation in DNA microarrays.

Experiments are described wherein hybridization takes place between the surface-bound sequences (referred to as *probes)* and the sequences in solution (*targets*) carrying a fluorophore. The amount of hybridized target is measured from the emitted fluorescence from a given location (spot) on the microarray surface. It is illustrated that, contrary to a widespread belief, in DNA microarrays relaxation times may largely exceed typical experimental times, causing a breakdown of thermal equilibrium. Experiments are performed on a commercial microarray platform under the same buffer conditions as in typical biological experiments. They are further corroborated by the analysis of an extended kinetic model. In equilibrium one expects that the intensity measured from a spot is described by the Langmuir model

$$ I = \frac{Ace^{\frac{\Delta G}{RT}}}{1+ce^{\frac{\Delta G}{RT}}} \approx Ace^{\frac{\Delta G}{RT}} \qquad [18] $$

where A sets the intensity scale, R is the gas constant, T is the temperature, c the target concentration and $\Delta G$ the hybridization free energy. It is to be noticed that a different sign convention is used compared to the first example. This is merely a matter of choosing the bound or the unbound state as the reference state for free energy differences. In Eq. [18] it took $ce^{\Delta G/RT}$ << 1 (weak binding and small concentrations), a limit which applies to the experiments discussed here.

The experimental setup is schematically shown in Table 6 and further similar to the first example.

Figure 11 shows a plot of $I/c$ vs. $\Delta\Delta G$ for four experiments at different concentrations using the setup of Table 6 (the target sequence is the 30-mer). $\Delta\Delta G \Xi \Delta G - \Delta G_{PM}$ is the difference in hybridization free energies between a given sequence and the perfect match (PM) sequence, calculated from the nearest-neighbour parameters.

The collapse of the $I/c$ vs. $\Delta\Delta G$ plots into a single curve shows that $I \propto c$ as expected from the low concentration limit of Eq. [18].

However, the dependence on $\Delta G$ is not in full agreement with Eq. [18]. In the regime deviating from Eq. [18] log I scales approximately linearly with $\Delta G$ but with a slope smaller than 1/RT.

Hybridization dynamics of oligonucleotides in aqueous solution is usually described as a two state process characterized by one association and one dissociation rate.

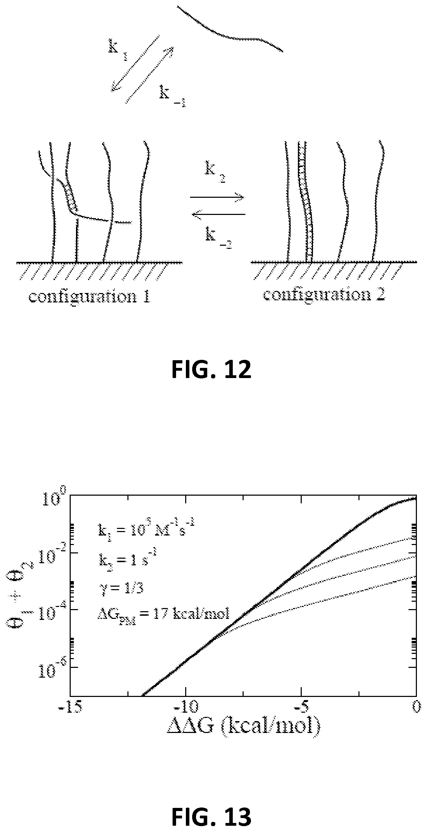

The Langmuir isotherm itself (Eq. [18]) is a two state model. Hybridization in DNA microarrays is likely to be more complex than a simple two state process. Probes are tethered to the surface by one end and can form a dense brush, which hinders and slows down hybridization. The typical distance between probes is of about 10 nanometers, and the length of a fully stretched 30-mer duplex is of 10 nm and its thickness of 2 nm. Probe sequences in the experiment have also a poly(A) 30-mer spacer (see Table 6). Therefore a single target molecule can interact with more than one probe. Taking this into account, we have extended the two state hybridization model with an additional intermediate state (FIG. 12). Indicating with $\theta_1$ and $\theta_2$, the fraction of partially and fully hybridized probes on the microarray, the kinetics of these reactions is given by

$$ \frac{d\theta_1}{dt} = ck_1\left(1 - \theta_1 - \theta_2\right) + k_{-2}\theta_{-2} - (k_{-1} + k_2)\theta_1 \qquad [19] $$

$$ \frac{d\theta_2}{dt} = k_2\theta_2 - k_{-2}\theta_2 \qquad [20] $$

where c is the target concentration in solution and $k_1$, $k_{-1}$, $k_2$ and $k_{-2}$ the four rates involved (see Fig. 12). For simplicity we have assumed that at most a single target molecule can bind to a given probe. The rate constants, using a two state model description, have been measured in several microarray experiments. The hybridization of a common target sequence to a perfect match probe and to a probe containing one mismatch were considered. One is interested in their dependence on ΔG. The following rates were used (at 45° C):

$$k_1^{(PM)} = 19 \cdot 10^{-4}\ M^{-1}\ s^{-1},$$

$$k_1^{(MM)} = 21 \cdot 10^{-4}\ M^{-1}\ s^{-1},$$

$$k_{-1}^{(PM)} = 12 \cdot 10^4\ s^{-1}\ \text{and}$$

$$k_{-1}^{(MM)} = 29 \cdot 10^4\ s^{-1}.$$

While there is more than a factor two of difference in the detachment rates, the attachment rates differ only by 10%. These results are in agreement with observation for kinetic behaviour in bulk solution. The probes in the experiment differ by at most by two nucleotides out of 30. It is assumed that both forward rates $k_1$ and $k_2$ are sequence independent. The reverse rates are then fixed by the thermodynamics relations

$$k_{-1} = k_1 e^{-\Delta G/RT}\ ;$$
$$k_{-2} = k_2 e^{-(\Delta G - \Delta G')/RT} \tag{21}$$

where ΔG' and ΔG are the free energy differences between configurations 1 and 2, and the unbound state, respectively. It is next assumed that ΔG', the free energy of the partially hybridized state, is monotonically dependent on ΔG. Moreover at unbinding (ΔG = 0), also ΔG' should vanish. As a simple approximation we then take

$$\Delta G' = \gamma \Delta G\ (\gamma < 1) \tag{22}$$

in order to approximate the expected monotonic dependence of ΔG' from ΔG. The model is then fully characterized by three parameters $k_1$, $k_2$ and $\gamma$.

FIG. 13 shows a plot of $\theta_1 + \theta_2$ vs. ΔΔG. These are obtained from the solution of Eqs. [19], [20] for different times and the following choice of parameters $k_1 = 10^5 M^{-1} s^{-1}$, $k_2 = 1\ s^{-1}$ and $\gamma = 1/3$ (for $k_1$ the value was used typically measured in kinetic experiments on microarrays, while $k_2$ and $\gamma$ are chosen to fit experimental data). In FIG. 13 thin solid lines are isotherms at finite times, while the thick line is the equilibrium isotherm (t → + ∞).

As time increases the equilibrium intensity is approached from below. To gain some more insight the limit of fast equilibration is considered for Eq. [19]. We then solve Eq. [20] using for $\theta_1$ its equilibrium value:

$$\theta_1^{(eq)} = \frac{ce^{\Delta G'/RT}}{c(e^{\Delta G/RT} + e^{\Delta G/RT}) + 1} \approx ce^{\Delta G'/RT} \tag{23}$$

where the low concentration limit $ce^{\Delta G/RT} \ll 1$ was taken. We have then

$$\theta_2(t) = ce^{\Delta G/RT}\ (1 - e^{-t/\tau}) \tag{24}$$

$$\tau^{-1} = k_{-2} = k_2 e^{-(1-\gamma)\Delta G/RT} \tag{25}$$

which is the inverse relaxation time. To get this Eqs. [21] and [23] were used in the limit $ce^{\Delta G/RT} \ll 1$.

The relaxation time depends on ΔG: weakly bounded sequences (small ΔG) equilibrate faster than strongly bounded ones (large ΔG). For fast equilibrating sequences (τ ≪ t) one recovers from Eq. [24] the usual Langmuir equilibrium; for sequences with long equilibration times τ ≫ t Eq. [24] is expanded to lowest order in t/τ. With this approximation we find that for a given time t

$$\theta_2(t) = \begin{cases} ce^{\Delta G/RT} & \Delta G \ll \Delta G^* \\ ctk_2 e^{\gamma \Delta G/RT} & \Delta G \gg \Delta G^* \end{cases} \tag{26}$$

where $\Delta G^*$ is a crossover free energy which depends on time and is obtained by setting $\tau = t$ in Eq. [25]. The measured intensity is $I = A(\theta_1 + \theta_2)$, however for any realistic choice of free energies $\theta_1 \ll \theta_2$, hence once can approximate $I \approx A\theta_2$. Equation [26] reproduces the two slopes in the log $I$ vs. $\Delta\Delta G$ plots as seen in the experiments.

It shows that the non-equilibrium regime is characterized by a slope equal to $\gamma/RT$. Turning now to experimental results, routinely, hybridization experiments are performed at constant temperature and buffer conditions for about 15h. Experiments were performed at shorter and longer hybridization times up to more than 86 h. Once the desired hybridization time has been reached the experiment is stopped, the microarray washed and scanned to measure the emitted fluorescence from every spot. Experiments at different hybridization times thus require different slides.

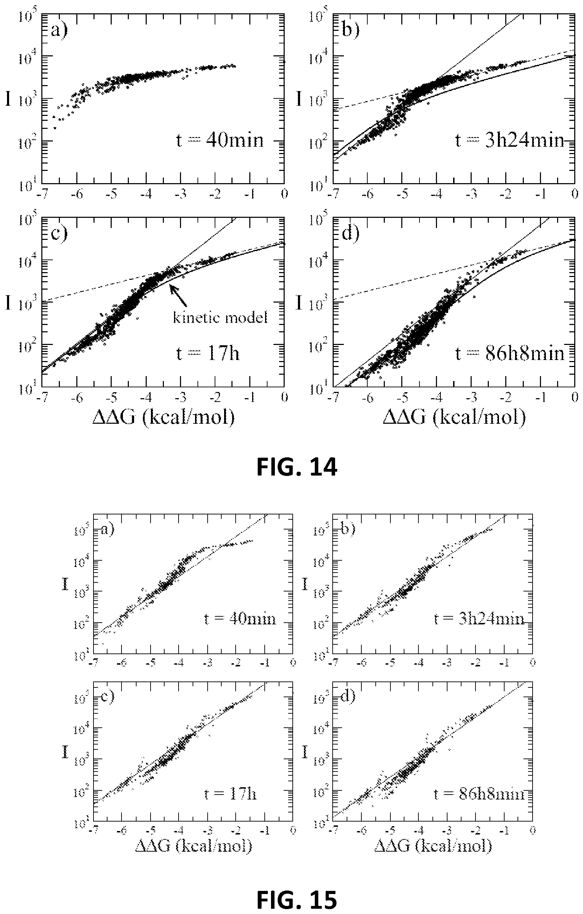

FIG. 14 shows a plot of $I$ vs. $\Delta\Delta G$ for a 30-mer target at four different times and for a concentration of 50 pM (the 17 h hybridization data are those already shown in Fig. 11). As the hybridization time increases a larger fraction of the data aligns along a line with a slope 1/RT over the full range of intensities, confirming that the observed deviations from the Langmuir model are due to the breakdown of thermodynamic equilibrium. Surprisingly, full equilibrium has not been reached here even after 85 hours. Apart from the shortest hybridization time the data are in agreement with the behaviour predicted by Eq. [26]. From the slope of the dashed line, using Eq. [26] it is estimated that $\gamma = 0.32$. As full hybridization involves a stretch of 30 nucleotides, this suggests that in the partially hybridized state the target and probe are bound for 10 nucleotides, the turn of an helix. The slope of the non-equilibrium regime of FIG. 14(a) is smaller than that of the dashed lines of FIG. 14(b,c,d). This is probably due to the protocol followed (this is the standard protocol): at time t = 0 both the solution containing the target molecules and the array are at room temperature.

They are then placed into an oven for the duration of the experiment at a constant temperature (T = 65° C for all experiments described in this letter). The slope is reminiscent of an initial "low" temperature hybridization.

When comparing FIG.14(b,c,d) one notices an overall decrease of the intensity scale, leading to a normalization of the constant $A$ in Eq. [18]. This is probably due to some degradation of the fluorophores (not surprising in view of the time span involved in the experiment). The solid lines in FIG. 14(b,c,d) are plots of the intensity $I = A(\theta_1 + \theta_2)$, where A was adjusted to match the global intensity scale. The agreement with the model is reasonable although not perfect.

Turning now to the case of hybridization to the shorter target sequence (25-mer, see Table. 6). Data are shown in FIG. 15. It is expected thatfaster equilibration for 25-mers occurs because the sequence has a lower $\Delta G$ and because for shorter target sequences it is expected that less entanglement occurs hence an increase of the $k_2$ rate. Both effects lead to smaller $\tau$ (see Eq. [25]). As can be seen in FIG. 15 the only deviation from the equilibrium isotherm are for the shortest hybridization time. The agreement with Eq. [18] is over three orders of magnitude in the intensity scale. Note an overall decrease of the intensity scale as observed in FIG. 14. The results are consistent with the idea that interaction of target molecule with multiple probes is responsible to the observed non-equilibrium behaviour. The present example shows that hybridization in DNA microarrays under standard conditions is characterized by a relaxation time which may largely exceed the experimental time. Since typical biological experiments involve target strands of 30-50 nucleotides, it is believed that the breakdown of equilibrium shown here on Agilent arrays, may occur in many different microarrays platforms and in biological experiments. It is found that in the non equilibrium regime the intensities are distributed according to an exponential distribution $e^{\gamma\Delta G/RT}$, with $\gamma < 1$. The breakdown of equilibrium carries important consequences: it lowers the specificity of the microarrays as devices for the detection of a desired sequence from a complex mixture. This can be illustrated with an example. Consider a probe at the microarray surface and two different sequences in solution: one perfect matching with the probe (at concentration $c_{PM}$) and one with a mismatch (at concentration $c_{MM}$). In the equilibrium regime the two sequences hybridize to the probe with probabilities proportional to $c_{PM}e^{\Delta G_{PM}/RT}$ and $c_{MM}e^{\Delta G_{MM}/RT}$, respectively. Assuming for simplicity equal target concentrations $c_{PM} = c_{MM}$ one has that the ratio of the two contributions is $e^{(\Delta G_{MM} - \Delta G_{PM})/RT} \approx 0.05$ where a typical value $\Delta G_{MM} - \Delta G_{PM} = 2$ kcal/mol and a temperature of $T = 65°$ C is used. In the non-equilibrium regime, due to the presence of a factor $\gamma = 1/3$ in the exponential the ratio is about 0.4. Therefore in the non-equilibrium regime a significant fraction of a measured signal may be due to hybridization to non-complementary targets a phenomenon known as cross-hybridization. For an optimal functioning of the microarrays it is then desirable to work under equilibrium conditions. Several parameters may influence the relaxation time as temperature, salt and buffer conditions. The experimental setup discussed provides a good test of equilibrium (single line vs. broken line in a $I$ vs. $\Delta\Delta G$ plot) and can be used to investigate the best working conditions for an optimal hybridization.

Other variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. Any reference signs in the claims should not be construed as limiting the scope.

The foregoing description details certain embodiments of the invention. It will be appreciated, however, that no matter how detailed the foregoing appears in text, the invention may be practiced in many ways, and is therefore not limited to the embodiments disclosed. It should be noted that the use of particular terminology when describing certain features or aspects of the invention should not be taken to imply that the terminology is being re-defined herein to be restricted to include any specific characteristics of the features or aspects of the invention with which that terminology is associated.

| Name | Sequence | Labeling |
|---|---|---|
| Target1 | 5' GTTTTCGAAGATTGGGTGGCACTGTTGTAA 3' | 20-mer poly A + Cy3 on 3' |
| Target2 | 5' CAGGGCCTCGTTATCAATGGAGTAGGTTTC 3' | 20-mer poly A + Cy3 on 3' |
| Target3 | 5' CTTTGTCGAGCTGGTATTTGGAGAACACGT 3' | 20-mer poly A + Cy3 on 3' |
| Target4 | 5' GCTTCTCCTTAATGTCACGCACGATTTCCC 3' | 20-mer poly A + Cy3 on 3' |

# TABLE 1

| No. of probes | Type of mismatch | Location of mismatch |
|---|---|---|
| 1 | Perfect match | — |
| 60 | Single mismatch (all three permutations) | site 6~25 |
| 945 | Double mismatch (all nine permutations) | site 6~25, separated by minimum five sites |

# TABLE 2

| Microarray | Experiment/target 1 | Experiment/target 2 | Experiment/target 3 | Experiment/target 4 |
|---|---|---|---|---|
| 1 | 1000 pM, a, f | 1000 pM, a, f | 1000 pM, a | 1000 pM, a |
| 2 | 750 pM, a, f | 500 pM, a, f | 500 pM, a | 500 pM, a |
| 3 | 500 pM, a, f | 100 pM, a, f | 100 pM, a | 100 pM, a |
| 4 | 250 pM, a, f | 50 pM, a, f | 50 pM, a | 50 pM, a |
| 5 | 100 pM, a, f | 1000 pM, b, f | 1000 pM, b | 1000 pM, b |
| 6 | 50 pM, a, f | 500 pM, b, f | 500 pM, b | 500 pM, b |
| 7 | 10 pM, a, f | 100 pM, b, f | 100 pM, b | 100 pM, b |
| 8 | 5 pM, a, f | 50 pM, b, f | 50 pM, b | 50 pM, b |

# TABLE 3

| X\Y | | A | C | G | T | | A | C | G | T | | A | C | G | T | | A | C | G | T |
|---|---|---|---|---|---|---|---|---|---|---|---|---|---|---|---|---|---|---|---|---|
| A | | 2.2 | 2.0 | 2.4 | 2.2 | | 3.0 | 2.8 | 3.0 | 3.0 | | 2.5 | 1.8 | 2.5 | 2.3 | | 2.4 | 2.2 | 2.4 | 2.3 |
| C | XAF YAF | 2.3 | 2.3 | 2.5 | 2.4 | XCF YCF | 3.0 | 2.8 | 3.0 | 3.0 | XGF YGF | 2.5 | 1.7 | 2.5 | 2.3 | XTF YTF | 2.4 | 2.2 | 2.4 | 2.5 |
| G | | 1.9 | 1.8 | 2.2 | 2.0 | | 2.7 | 2.5 | 2.7 | 2.7 | | 2.4 | 1.6 | 2.4 | 2.0 | | 2.0 | 1.8 | 2.1 | 2.3 |
| T | | 2.2 | 2.1 | 2.5 | 2.3 | | 3.1 | 2.9 | 3.1 | 3.1 | | 2.4 | 1.7 | 2.5 | 2.3 | | 2.4 | 2.2 | 2.4 | 2.5 |
| A | | 3.9 | 3.4 | 3.4 | 4.0 | | 2.5 | 2.4 | 2.4 | 2.8 | | 1.5 | 1.3 | 1.7 | 1.7 | | 2.4 | 1.8 | 2.3 | 1.9 |
| C | XCF YCF | 3.4 | 3.9 | 2.9 | 3.5 | XTF YTF | 2.4 | 2.3 | 2.3 | 2.7 | XAF YAF | 1.7 | 1.6 | 1.9 | 1.9 | XGF YGF | 2.7 | 2.1 | 2.6 | 2.2 |
| G | | 3.1 | 2.7 | 2.7 | 3.2 | | 2.5 | 2.5 | 2.3 | 2.8 | | 1.1 | 0.9 | 1.3 | 1.3 | | 2.5 | 1.9 | 2.4 | 2.0 |
| T | | 3.8 | 3.4 | 3.3 | 3.9 | | 2.5 | 2.5 | 2.4 | 2.8 | | 1.7 | 1.6 | 2.0 | 2.0 | | 2.8 | 2.2 | 2.7 | 2.3 |
| A | | 2.0 | 1.8 | 1.9 | 1.9 | | 3.5 | 3.6 | 3.1 | 3.3 | | 2.2 | 2.2 | 2.0 | 2.4 | | 2.3 | 2.4 | 2.0 | 2.2 |
| C | XGF YGF | 1.6 | 1.4 | 1.5 | 1.5 | XTF YCF | 3.2 | 3.3 | 2.8 | 3.0 | XTF YAF | 2.3 | 2.3 | 2.1 | 2.5 | XTF YTF | 2.1 | 2.2 | 1.7 | 2.0 |
| G | | 1.8 | 1.7 | 1.8 | 1.7 | | 3.1 | 3.2 | 2.8 | 2.9 | | 2.4 | 2.4 | 2.2 | 2.6 | | 2.4 | 2.5 | 2.1 | 2.4 |
| T | | 1.6 | 1.4 | 1.6 | 1.5 | | 3.2 | 3.3 | 2.9 | 3.0 | | 2.3 | 2.3 | 2.1 | 2.5 | | 2.2 | 2.3 | 1.9 | 2.1 |

# Table 4

| X\Y | | A | C | G | T | | A | C | G | T | | A | C | G | T | | A | C | G | T |
|---|---|---|---|---|---|---|---|---|---|---|---|---|---|---|---|---|---|---|---|---|
| A | | 3.3 | 2.9 | 2.3 | 2.0 | | 2.9 | 3.6 | 3.5 | 2.6 | | 2.3 | 1.7 | 2.9 | 1.8 | | 1.4 | 1.8 | 2.1 | 1.8 |
| C | XAF YAF | 3.6 | 3.3 | 2.6 | 2.2 | XCF YCF | 3.5 | 4.2 | 4.1 | 3.2 | XGF YGF | 2.7 | 2.0 | 3.2 | 2.1 | XTF YTF | 2.2 | 2.6 | 3.0 | 2.6 |
| G | | 3.0 | 2.3 | 2.6 | 2.3 | | 3.1 | 3.8 | 3.7 | 2.9 | | 2.6 | 2.0 | 3.2 | 2.1 | | 2.1 | 2.5 | 2.9 | 2.5 |
| T | | 3.1 | 1.8 | 2.1 | 1.8 | | 3.0 | 3.7 | 3.6 | 2.7 | | 2.5 | 1.8 | 3.0 | 1.9 | | 1.9 | 2.3 | 2.6 | 2.3 |
| A | | 3.4 | 4.3 | 4.4 | 4.5 | | 3.2 | 2.5 | 2.3 | 2.2 | | 0.8 | 0.8 | 1.3 | 1.0 | | 2.1 | 1.8 | 2.6 | 1.7 |
| C | XCF YCF | 3.6 | 4.5 | 4.5 | 4.7 | XTF YTF | 2.7 | 3.0 | 2.8 | 2.7 | XAF YAF | 1.6 | 1.5 | 2.1 | 1.7 | XGF YGF | 2.8 | 2.2 | 3.2 | 2.4 |
| G | | 3.1 | 4.0 | 4.1 | 4.2 | | 2.3 | 2.6 | 2.4 | 2.4 | | 0.7 | 0.7 | 1.1 | 0.8 | | 2.1 | 1.5 | 2.6 | 1.7 |
| T | | 2.9 | 3.3 | 3.6 | 3.7 | | 2.1 | 2.4 | 2.2 | 2.2 | | 1.4 | 1.3 | 1.8 | 1.5 | | 2.3 | 1.7 | 2.8 | 1.9 |
| A | | 2.0 | 1.7 | 1.7 | 1.7 | | 3.3 | 3.6 | 3.6 | 3.1 | | 2.4 | 2.2 | 2.4 | 2.5 | | 2.5 | 2.8 | 2.4 | 2.6 |
| C | XGF YGF | 1.6 | 1.3 | 1.3 | 1.3 | XTF YCF | 3.6 | 4.0 | 4.0 | 3.4 | XTF YAF | 2.5 | 2.2 | 2.4 | 2.6 | XTF YTF | 2.3 | 2.6 | 2.2 | 2.4 |
| G | | 1.4 | 1.1 | 1.1 | 1.1 | | 3.6 | 3.9 | 3.9 | 3.4 | | 2.6 | 2.4 | 2.6 | 2.7 | | 2.4 | 2.8 | 2.4 | 2.6 |
| T | | 1.4 | 1.1 | 1.1 | 1.1 | | 3.2 | 3.6 | 3.6 | 3.0 | | 2.5 | 2.2 | 2.4 | 2.6 | | 1.8 | 2.2 | 1.7 | 2.0 |

# Table 5

| Target sequences in solution |
|---|
| 1.  CTTTGTCGAGCTGGTATTTGGAGAACACGT |
| 2.      TCGAGCTGGTATTTGGAGAACACGT |

| Probes at the microarray surface | |
|---|---|
| PM | ACGTGTTCTCCAAATACCAGCTCGACAAAG |
| | ACGTATTCTCCAAATACCAGCTCGACAAAG |
| 1MM | ACGTCTTCTCCAAATACCAGCTCGACAAAG |
| | ACGTTTTCTCCAAATACCAGCTCGACAAAG |
| | ... |
| 2MM | ACGTATTCTACAAATACCAGCTCGACAAAG |
| | ... |

# TABLE 6

**Claims**

1.  A method for analysing hybridisation between a target in a sample solution and a probe bound at a surface, the method comprising receiving detection intensity results for hybridisation of the target with a plurality of different probes, the probes being selected so that a range of hybridisation detection intensity results for the hybridisation between the target and the probe is covered, and analysing the detection intensity results as function of hybridisation free energy, wherein the hybridisation free energy for the hybridisation between a target in solution and a probe bound at a surface is determined based on a model, the model being a model wherein a thermodynamic non-equilibrium state for the hybridisation is taken into account and wherein analysing the detection intensity results as function of the hybridisation free energy comprises analysing the logarithm of the detection intensity results as function hybridisation free energy for a range of hybridisation free energies and determining whether one linear relationship or more linear relationships can be distinguished between parts of the logarithm of the detection intensity results and the hybridisation free energy.

2. A method according to claim 1, wherein for said receiving detection intensity results or for said analysing reaching of thermodynamic equilibrium is taken into consideration.

3. A method according to claim 2, wherein said receiving detection intensity results taking into consideration reaching of thermodynamic equilibrium comprises receiving detection intensity results for hybridisation of the target with a plurality of different probes obtained under hybridisation conditions wherein thermodynamic equilibrium has been reached.

4. A method according to claim 3, wherein said hybridisation conditions comprise one or a combination of hybridisation time, probe length or temperature.

5. A method according to any of the previous claims, wherein said analyzing comprises deriving that the hybridisation has not reached equilibrium when more than one linear relationship can be distinguished between parts of the logarithm of the detection intensity results and the hybridisation free energy.

6. A method according to any of the previous claims, wherein said determining the hybridisation free energy for the hybridisation between a target in solution and a probe bound at a surface is based on a nearest-neighbour model.

7. A method according to any of claims 1 to 6, wherein for a given target a perfect match probe, and probes with up to two nucleotide non-complementary elements are provided, each to separate microarray spots for interaction with the target during the hybridisation, and analysing the detection intensity results as function of the hybridisation free energy comprises determining a set of free energy parameters of a nearest neighbour model for the hybridisation free energy based on said hybridization microarrayexperiment.

8. A method according to any of the previous claims, wherein analysing the detection intensity results as function of the hybridisation free energy comprises determining whether measured detection intensity results correspond with thermodynamic equilibrium for the hybridisation.

9. A method according to any of the previous claims, wherein receiving detection intensity results comprises receiving detection intensity results for hybridisation between the plurality of different probes and the actual target, the probes being selected so that a range of hybridization detection intensities for the hybridisation between each possible target of the set of known possible targets and the probe is covered, and analysing the detection intensity results as function of the hybridisation free energy comprises analysing the detection intensity results as function of the hybridisation free energy for each of the possible targets of the set of known possible targets and deriving the presence or concentration of an actual target by detecting a predetermined relationship between the detection intensity results and the hybridisation free energy.

10. A method according to claim 9, wherein the actual target is a minority target and wherein the set of known possible targets comprises, besides the minority target, also a main target differing from the minority target in one or two noncomplementary elements, or wherein the actual target is an unknown target differing from a main target in one or two non-complementary elements, and wherein the set of known possible targets comprises the main target and a set of targets differing from the main target in one or two non-complementary elements.

11. A method according to any of the previous claims, for use in detecting single nucleotide polymorph isms.

12. A method according to any of the previous claims, wherein hybridisation is performed in a microarray.

13. A system for analysing hybridisation between a target in a sample solution and a probe bound at a surface, the system comprising a receiving means adapted for receiving detection intensity results for hybridisation of the target with a plurality of different probes, the probes being selected so that a range of detection intensities for the hybridization between the target and the probe is covered, and an analysing means adapted for analysing the detection intensity results as function of the hybridisation free energy, wherein the analysing means is adapted for determining the hybridisation free energy for the hybridisation between a target in solution and a probe bound at a surface based on a model, the model being a model wherein a thermodynamic non-equilibrium state for the hybridisation is taken into account, and wherein the analysing means is adapted for analysing the logarithm of the detection intensity results as function hybridisation free energy for a range of hybridisation free energies and for determining whether one linear relationship or more linear relationships can be distinguished between parts of the logarithm of the detection intensity results and the hybridisation free energy.

14. Use of a method for analysing hybridisation according to any of claims 1 to 12 for designing a hybridisation kit, the hybridisation kit comprising a microarray having a plurality of microarray spots each of them comprising a probe, the plurality of different probes being selected so that the corresponding hybridisation covers a range of hybridisation detection intensities for the hybridisation of each possible target of the set of known possible targets.

15. A computer program product adapted for performing, when executed on a computing device, a method for analysing hybridisation as described in any of claims 1 to 12.

**Patentansprüche**

1. Verfahren zum Analysieren von Hybridisierung zwischen einem Ziel in einer Probenlösung und einer an eine Oberfläche gebundenen Sonde, wobei das Verfahren umfasst:

   - Empfangen von Nachweisintensitätsergebnissen für die Hybridisierung des Ziels mit einer Vielzahl verschiedener Sonden, wobei die Sonden so ausgewählt sind, dass ein Bereich von Hybridisierungs-Nachweisintensitätsergebnissen für die Hybridisierung zwischen dem Ziel und der Sonde abgedeckt wird, und
   - Analysieren der Nachweisintensitätsergebnisse als Funktion der freien Energie der Hybridisierung,

   wobei die freie Energie der Hybridisierung für die Hybridisierung zwischen einem Ziel in Lösung und einer an eine Oberfläche gebundene Sonde auf der Grundlage eines Modells bestimmt wird, wobei das Modell ein Modell ist, bei dem ein thermodynamischer Nichtgleichgewichtszustand für die Hybridisierung berücksichtigt wird, und wobei die Analyse der Nachweisintensitätsergebnisse als Funktion der freien Energie der Hybridisierung die Analyse des Logarithmus der Nachweisintensitätsergebnisse als Funktion der freien Energie der Hybridisierung für einen Bereich von freien Energien der Hybridisierung und die Bestimmung, ob eine lineare Beziehung oder mehrere lineare Beziehungen zwischen Teilen des Logarithmus der Nachweisintensitätsergebnisse und der freien Energie der Hybridisierung unterschieden werden können, umfasst.

2. Verfahren gemäß Anspruch 1, wobei für das Empfangen der Nachweisintensitätsergebnisse oder für die Analyse das Erreichen von thermodynamischem Gleichgewicht in Betracht gezogen wird.

3. Verfahren gemäß Anspruch 2, wobei das Empfangen der Nachweisintensitätsergebnisse unter Betrachtung des Erreichens von thermodynamischem Gleichgewicht das Empfangen von Nachweisintensitätsergebnissen für Hybridisierung des Ziels mit einer Vielzahl verschiedener Sonden, erhalten unter Hybridisierungsbedingungen, wobei thermodynamisches Gleichgewicht erreicht worden ist, umfasst.

4. Verfahren gemäß Anspruch 3, wobei die Hybridisierungsbedingungen eines oder eine Kombination von Hybridisierungszeit, Sondenlänge und Temperatur umfassen.

5. Verfahren gemäß einem der vorstehenden Ansprüche, wobei das Analysieren das Ableiten umfasst, dass die Hybridisierung kein Gleichgewicht erreicht hat, wenn mehr als eine lineare Beziehung zwischen Teilen des Logarithmus der Nachweisintensitätsergebnisse und der freien Energie der Hybridisierung unterschieden werden kann.

6. Verfahren gemäß einem der vorstehenden Ansprüche, wobei das Bestimmen der freien Energie der Hybridisierung für die Hybridisierung zwischen einem Ziel in Lösung und einer an eine Oberfläche gebundenen Sonde auf der Grundlage eines nächster-Nachbar-Modells steht.

7. Verfahren gemäß einem der Ansprüche 1 bis 6, wobei

- für ein gegebenes Ziel eine perfekt passende Sonde und Sonden mit bis zu zwei nichtkomplementären Nukleotidelementen bereitgestellt werden, jeweils an getrennten Mikroarray-Flecken für die Wechselwirkung mit dem Ziel während der Hybridisierung, und
- das Analysieren der Nachweisintensitätsergebnisse als Funktion der freien Energie der Hybridisierung das Bestimmen eines Satzes von Parametern der freien Energie eines nächster-Nachbar-Modells für die freie Energie der Hybridisierung auf der Grundlage des Hybridisierungs-Mikroarray-Experiments umfasst.

8. Verfahren gemäß einem der vorstehenden Ansprüche, wobei das Analysieren der Nachweisintensitätsergebnisse als Funktion der freien Energie der Hybridisierung die Bestimmung umfasst, ob gemessene Nachweisintensitätsergebnisse thermodynamischem Gleichgewicht für die Hybridisierung entsprechen.

9. Verfahren gemäß einem der vorstehenden Ansprüche, wobei

- das Empfangen von Nachweisintensitätsergebnissen das Empfangen von Nachweisintensitätsergebnissen für die Hybridisierung zwischen der Vielzahl verschiedener Sonden und dem tatsächlichen Ziel umfasst, wobei die Sonden so ausgewählt sind, dass ein Bereich von Hybridisierungs-Nachweisintensitäten für die Hybridisierung zwischen jedem möglichen Ziel des Satzes von bekannten möglichen Zielen und der Sonde abgedeckt wird, und
- das Analysieren der Nachweisintensitätsergebnisse als Funktion der freien Energie der Hybridisierung das Analysieren der Nachweisintensitätsergebnisse als Funktion der freien Energie der Hybridisierung für jedes der möglichen Ziele des Satzes von bekannten möglichen Zielen und Ableiten des Vorhandenseins oder der Konzentration eines tatsächlichen Ziels durch Nachweisen einer vorbestimmten Beziehung zwischen den Nachweisintensitätsergebnissen und der freien Energie der Hybridisierung umfasst.

10. Verfahren gemäß Anspruch 9, wobei das tatsächliche Ziel ein Minoritätsziel ist und wobei der Satz von bekannten möglichen zielen neben dem Minoritätsziel auch ein Hauptziel umfasst, das sich in einem oder zwei nichtkomplementären Elementen von dem Minoritätsziel unterscheidet, oder wobei das tatsächliche Ziel ein unbekanntes Ziel ist, das sich in einem oder zwei nichtkomplementären Elementen von einem Hauptziel unterscheidet, und wobei der Satz von bekannten möglichen Zielen das Hauptziel und einen Satz von Zielen umfasst, die sich in einem oder zwei nichtkomplementären Elementen von dem Hauptziel unterscheiden.

11. Verfahren gemäß einem der vorstehenden Ansprüche für die Verwendung bei dem Nachweisen von Einzelnukleotid-Polymorphien.

12. Verfahren gemäß einem der vorstehenden Ansprüche, wobei die Hybridisierung in einem Mikroarray durchgeführt wird.

13. System zum Analysieren von Hybridisierung zwischen einem Ziel in einer Probenlösung und einer an eine Oberfläche gebundenen Sonde, wobei das System umfasst:

- ein Empfangsmittel, ausgelegt zum Empfangen von Nachweisintensitätsergebnissen für die Hybridisierung des Ziels mit einer Vielzahl verschiedener Sonden, wobei die Sonden so ausgewählt sind, dass ein Bereich von Nachweisintensitäten für die Hybridisierung zwischen dem Ziel und der Sonde abgedeckt wird, und
- ein Analysemittel, ausgelegt zum Analysieren der Nachweisintensitätsergebnisse als Funktion der freien Energie der Hybridisierung,

wobei das Analysemittel dafür ausgelegt ist, die freie Energie der Hybridisierung für die Hybridisierung zwischen einem Ziel in Lösung und einer an eine Oberfläche gebundenen Sonde auf der Grundlage eines Modells zu bestimmen, wobei das Modell ein Modell ist, bei dem ein thermodynamischer Nichtgleichgewichtszustand für die Hybridisierung berücksichtigt wird, und
wobei das Analysemittel dafür ausgelegt ist, den Logarithmus der Nachweisintensitätsergebnisse als Funktion der freien Energie der Hybridisierung für einen Bereich von freien Energien der Hybridisierung zu analysieren und zu bestimmen, ob eine lineare Beziehung oder mehrere lineare Beziehungen zwischen Teilen des Logarithmus der Nachweisintensitätsergebnisse und der freien Energie der Hybridisierung unterschieden werden können.

14. Verwendung eines Verfahrens zum Analysieren von Hybridisierung gemäß einem der Ansprüche 1 bis 12 zum Entwickeln eines Hybridisierungskits, wobei das Hybridisierungskit umfasst:

ein Mikroarray mit einer Vielzahl von Mikroarray-Flecken, von denen jeder eine Sonde umfasst, wobei die Vielzahl verschiedener Sonden so ausgewählt ist, dass die entsprechende Hybridisierung einen Bereich von Hybridisierungs-Nachweisintensitäten für die Hybridisierung jedes möglichen Ziels des Satzes bekannter möglicher Ziele abdeckt.

**15.** Computerprogrammprodukt, dafür ausgelegt, bei Ausführung auf einer Computervorrichtung ein Verfahren zum Analysieren von Hybridisierung gemäß einem der Ansprüche 1 bis 12 durchzuführen.

## Revendications

**1.** Procédé d'analyse d'hybridation entre une cible dans une solution d'échantillon et une sonde liée à une surface, le procédé comprenant

  - la réception de résultats d'intensité de détection pour hybridation de la cible avec une pluralité de sondes différentes, les sondes étant choisies de sorte qu'une plage de résultats d'intensité de détection d'hybridation pour l'hybridation entre la cible et la sonde soit couverte, et
  - l'analyse des résultats d'intensité de détection en fonction de l'énergie libre d'hybridation,

dans lequel l'énergie libre d'hybridation pour l'hybridation entre une cible en solution et une sonde liée à une surface est déterminée sur la base d'un modèle, le modèle étant un modèle dans lequel un état de non-équilibre thermodynamique pour l'hybridation est pris en compte et
dans lequel l'analyse des résultats d'intensité de détection en fonction de l'énergie libre d'hybridation comprend l'analyse du logarithme des résultats d'intensité de détection en fonction de l'énergie libre d'hybridation pour une plage d'énergies libres d'hybridation et la détermination du fait qu'une relation linéaire ou plusieurs relations linéaires peuvent être distinguées ou non entre des parties du logarithme des résultats d'intensité de détection et l'énergie libre d'hybridation.

**2.** Procédé selon la revendication 1, dans lequel, pour ladite réception de résultats d'intensité de détection ou pour ladite analyse, l'atteinte d'un équilibre thermodynamique est prise en compte.

**3.** Procédé selon la revendication 2, dans lequel ladite réception de résultats d'intensité de détection prenant en compte l'atteinte d'un équilibre thermodynamique comprend la réception de résultats d'intensité de détection pour l'hybridation de la cible avec une pluralité de sondes différentes obtenues dans des conditions d'hybridation dans lesquelles l'équilibre thermodynamique a été atteint.

**4.** Procédé selon la revendication 3, dans lequel lesdites conditions d'hybridation comprennent l'un ou une combinaison de temps d'hybridation, de longueur de sonde ou de température.

**5.** Procédé selon l'une quelconque des revendications précédentes, dans lequel ladite analyse comprend la déduction que l'hybridation n'a pas atteint l'équilibre lorsque plus d'une relation linéaire peut être distinguée entre des parties du logarithme des résultats d'intensité de détection et l'énergie libre d'hybridation.

**6.** Procédé selon l'une quelconque des revendications précédentes, dans lequel ladite détermination de l'énergie libre d'hybridation pour l'hybridation entre une cible en solution et une sonde liée à une surface est basée sur un modèle du voisin le plus proche.

**7.** Procédé selon l'une quelconque des revendications 1 à 6, dans lequel

  - pour une cible donnée, une sonde de correspondance parfaite, et des sondes avec jusqu'à deux éléments nucléotidiques non complémentaires sont fournies, chacune à des points de microréseau séparés pour interaction avec la cible pendant l'hybridation, et
  - l'analyse des résultats d'intensité de détection en fonction de l'énergie libre d'hybridation comprend la détermination d'un ensemble de paramètres d'énergie libre d'un modèle de voisin le plus proche pour l'énergie libre d'hybridation sur la base dudit essai d'hybridation sur microréseau.

**8.** Procédé selon l'une quelconque des revendications précédentes, dans lequel l'analyse des résultats d'intensité de détection en fonction de l'énergie libre d'hybridation comprend la détermination du fait que les résultats d'intensité de détection mesurés correspondent ou non à un équilibre thermodynamique pour l'hybridation.

9. Procédé selon l'une quelconque des revendications précédentes, dans lequel

- la réception de résultats d'intensité de détection comprend la réception de résultats d'intensité de détection pour l'hybridation entre la pluralité de sondes différentes et la cible réelle, les sondes étant choisies de sorte qu'une plage d'intensités de détection d'hybridation pour l'hybridation entre chaque cible possible de l'ensemble de cibles possibles connues et la sonde soit couverte, et
- l'analyse des résultats d'intensité de détection en fonction de l'énergie libre d'hybridation comprend l'analyse des résultats d'intensité de détection en fonction de l'énergie libre d'hybridation pour chacune des cibles possibles de l'ensemble de cibles possibles connues et la déduction de la présence ou de la concentration d'une cible réelle par détection d'une relation prédéterminée entre les résultats d'intensité de détection et l'énergie libre d'hybridation.

10. Procédé selon la revendication 9, dans lequel la cible réelle est une cible minoritaire et dans lequel l'ensemble de cibles possibles connues comprend, en dehors de la cible minoritaire, également une cible principale différant de la cible minoritaire dans un ou deux éléments non complémentaires, ou dans lequel la cible réelle est une cible inconnue différant d'une cible principale dans un ou deux éléments non complémentaires, et dans lequel l'ensemble de cibles possibles connues comprend la cible principale et un ensemble de cibles différant de la cible principale dans un ou deux éléments non complémentaires.

11. Procédé selon l'une quelconque des revendications précédentes, pour utilisation dans la détection de polymorphismes de nucléotide unique.

12. Procédé selon l'une quelconque des revendications précédentes, dans lequel l'hybridation est effectuée dans un microréseau.

13. Système d'analyse d'hybridation entre une cible dans une solution d'échantillon et une sonde liée à une surface, le système comprenant

- un moyen de réception adapté pour la réception de résultats d'intensité de détection pour l'hybridation de la cible avec une pluralité de sondes différentes, les sondes étant choisies de sorte qu'une plage d'intensités de détection pour l'hybridation entre la cible et la sonde soit couverte, et
- un moyen d'analyse adapté pour analyser les résultats d'intensité de détection en fonction de l'énergie libre d'hybridation,

dans lequel le moyen d'analyse est adapté pour déterminer l'énergie libre d'hybridation pour l'hybridation entre une cible en solution et une sonde liée à une surface sur la base d'un modèle, le modèle étant un modèle dans lequel un état de non-équilibre thermodynamique pour l'hybridation est pris en compte, et

dans lequel le moyen d'analyse est adapté pour analyser le logarithme des résultats d'intensité de détection en fonction de l'énergie libre d'hybridation pour une plage d'énergies libres d'hybridation et pour déterminer si une relation linéaire ou plusieurs relations linéaires peuvent être distinguées entre des parties du logarithme des résultats d'intensité de détection et l'énergie libre d'hybridation.

14. Utilisation d'un procédé pour analyser l'hybridation selon l'une quelconque des revendications 1 à 12 pour concevoir un kit d'hybridation, le kit d'hybridation comprenant un microréseau ayant une pluralité de points de microréseau dont chacun comprend une sonde, la pluralité de sondes différentes étant choisies de sorte que l'hybridation correspondante couvre une plage d'intensités de détection d'hybridation pour l'hybridation de chaque cible possible de l'ensemble de cibles possibles connues.

15. Produit de programme informatique adapté pour conduire, lorsqu'il est exécuté sur un dispositif informatique, un procédé d'analyse de l'hybridation tel que décrit dans l'une quelconque des revendications 1 à 12.

**FIG. 1**

**FIG. 2**

hypothetical target = true target

$-\Delta\Delta G_{\mu\ array}$ (kcal/mol)

## FIG. 3A

hypothetical target = true target + a mismatch

$-\Delta\Delta G_{\mu\ array}$ (kcal/mol)

## FIG. 3B

FIG. 4

500

Receiving measured intensities for a hybridisation experiment using a plurality of probes having a different hybridisation free energy ΔG for at least one target

510

Evaluating measured intensities as function of the hybridization free energy ΔG for at least one target for a range of hybridization free energies

520

Deriving information regarding the at least one target and/or its hybridization from said evaluation

530

## FIG. 5

**FIG. 6**

**FIG. 7**

**FIG. 8**

**FIG. 9**

**FIG. 10**

**FIG. 11**

**FIG. 12**

**FIG. 13**

FIG. 14

FIG. 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **FISH et al.** *Nucleic Acids Research,* 2007, vol. 35 (21), 7197-7208

- **WEI et al.** *Nucleic Acids Research,* 2005, vol. 33 (8), e78